# EUROPEAN PATENT APPLICATION

(11) **EP 4 628 014 A2**
(43) Date of publication of application: **08.10.2025**
(21) Application number: 25177419.6
(22) Date of filing: 05.02.2019
(51) Int. Cl.: A61B 5/145

(54) **NOTES AND EVENT LOG INFORMATION ASSOCIATED WITH ANALYTE SENSORS**

(30) Priority: 05.02.2018 US 201862626410 P
(62) Divisional of application: 19708698.6
(71) Applicant: Abbott Diabetes Care, Inc., Alameda, CA 94502 (US)
(72) Inventor: KUMAR, Ashwin, Oakland, CA 94618 (US); GOLDSMITH, Joel, Oakland, CA 94502 (US)
(74) Representative: Mathys & Squire

(57) **Abstract**

The detection of various analytes within an individual can sometimes be vital for monitoring the condition of their health. Deviation from normal analyte levels can be indicative of a number of physiological conditions. Improved computing devices that allow a user to input data about the sensor user's lifestyle and a user to access an event log associated with an analyte monitoring sensor may be beneficial.

## Description

### CROSS-REFERENCE TO RELATED APPLICATIONS

The present application claims the benefit of priority under 35 U.S.C. § 119 from U.S. Provisional Patent Application 62/626,410, filed on February 5, 2018 and incorporated by reference herein in its entirety.

### STATEMENT REGARDING FEDERALLY SPONSORED RESEARCH OR DEVELOPMENT

Not applicable.

### BACKGROUND

The detection of various analytes within an individual can sometimes be vital for monitoring the condition of their health. Deviation from normal analyte levels can be indicative of a number of physiological conditions. In diabetic individuals, for example, detection of abnormal glucose levels can be essential for maintaining good health. By monitoring glucose levels with sufficient regularity, a diabetic individual may be able to take corrective action (e.g., by injecting insulin to lower glucose levels or by eating to raise glucose levels) before significant physiological harm occurs. Other analytes subject to physiological dysregulation may be similarly desirable to monitor in order to maintain good health.

Analyte monitoring in an individual may take place periodically or continuously over a period of time. Periodic analyte monitoring may take place by withdrawing a sample of bodily fluid, such as blood, at set time intervals and analyzing *ex vivo.* Continuous analyte monitoring may be conducted using one or more sensors that remain implanted within a tissue of an individual, such as dermally, subcutaneously, or intravenously, though which analyses may take place *in vivo.* Implanted sensors may collect analyte data continuously or sporadically, depending on an individual's particular health needs.

An individual's analyte levels may be affected by various external stimuli related to that individual's particular lifestyle. For example, if the individual is diabetic, that individual's food intake, exercise, or injection of insulin will affect their glucose levels. Such lifestyle actions may additionally affect other analyte levels. Moreover, other individual-specific lifestyle events may affect analyte levels and/or be valuable for an individual to monitor to determine what lifestyle events influence their analyte levels.

Conventionally, analyte sensors provide feedback to a user based upon information (e.g., data) collected by the sensor via a receiver, which may limit a user's ability to input lifestyle data, particularly that which may impact an output of the analyte sensor. Further, errors or events encountered during operation of the receiver and/or sensor may be inaccessible or difficult to access by the user and/or by trouble shooting personnel (e.g., customer service personnel). As such, erratic analyte measurements without a known source or cause may result.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIGS. 1A and 1B show display screens of a computing device presenting an analyte monitoring scan display window that are compatible with one or more embodiments of the present disclosure.
FIGS. 2A to 2C show display screens of a computing device presenting an input display window that are compatible with one or more embodiments of the present disclosure
FIGS. 3A to 3J show a series of input display window views depicting user interaction therewith that are compatible with one or more embodiments of the present disclosure.
FIGS. 4A to 4J show a series of input display window views depicting user interaction therewith that are compatible with one or more embodiments of the present disclosure.
FIGS. 5A and 5B show display screens of a computing device presenting an analyte monitoring scan display window after user input that are compatible with one or more embodiments of the present disclosure.
FIGS. 6A to 6D show display screens of a computing device presenting an analyte monitoring daily display window that are compatible with one or more embodiments of the present disclosure.
FIGS. 7A to 7C show display screens of a computing device presenting a pop-up display window that are compatible with one or more embodiments of the present disclosure.
FIGS. 8A and 8B show display screens of a computing device presenting various user selections to generate a report that are compatible with one or more embodiments of the present disclosure.
FIGS. 9A and 9B show display screens of a computing device presenting a limited number of user selections, including an event log button, that are compatible with one or more embodiments of the present disclosure.
FIGS. 10A and 10B show display screens of a computing device presenting an event log display window that are compatible with one or more embodiments of the present disclosure.
FIG. 11 is a block diagram depicting an example of an in vivo analyte monitoring system that is compatible with one or more embodiments of the present disclosure.
FIG. 12 is a block diagram depicting an example of a data processing unit that is compatible with one or more embodiments of the present disclosure.
FIG. 13 is a block diagram depicting an example of a display device that is compatible with one or more embodiments of the present disclosure.
FIG. 14 as a schematic diagram depicting an example of an analyte sensor that is compatible with one or more embodiments of the present disclosure.
FIG. 15A is a perspective view depicting an example embodiment of an analyte sensor penetrating through the skin that is compatible with one or more embodiments of the present disclosure.
FIG. 15B is a cross sectional view depicting a portion of the analyte sensor of FIG. 15A that is compatible with one or more embodiments of the present disclosure.
FIGS. 15C and 15D show a plan view of a transcutaneous sensor that is compatible with one or more embodiments of the present disclosure.
FIGS. 16-19 are cross-sectional views depicting examples of analyte sensors that are compatible with one or more embodiments of the present disclosure.
FIG. 20A is a cross-sectional view depicting an example of an analyte sensor that is compatible with one or more embodiments of the present disclosure.
FIGS. 20B-20C are cross-sectional views depicting examples of analyte sensors as viewed from line A-A of FIG. 20A that are compatible with one or more embodiments of the present disclosure.
FIG. 21 is a conceptual view depicting an example of an analyte monitoring system that is compatible with one or more embodiments of the present disclosure.
FIG. 22 is a block diagram depicting an example of on body electronics that is compatible with one or more embodiments of the present disclosure.
FIG. 23 is a block diagram depicting an example of a display device that is compatible with one or more embodiments of the present disclosure.
FIG. 24 is a flow diagram depicting an example of information exchange within an analyte monitoring system that is compatible with one or more embodiments of the present disclosure.
FIGS. 25A and 25B show display screens of a computing device presenting a start-up display window that are compatible with one or more embodiments of the present disclosure.

### DETAILED DESCRIPTION

The present disclosure generally relates to computing devices that allow a user to input data about a sensor user's lifestyle and a user to access an event log associated with an analyte monitoring sensor.

The computing devices described in the present disclosure allow improved user interaction therewith by allowing the user to customize inputs related to a sensor user's lifestyle and easily and quickly access such information, particularly as it relates to specific analyte levels occurring in the body of the sensor user. As used herein, the term "user," and grammatical variants thereof, with reference to use of the computer devices and display windows of the present disclosure includes any individual that manipulates the computing device and interacts with the display screens thereof, including, but not limited to, a sensor user, the physician of a sensor user, loved ones of a sensor user, and the like. The term "sensor user," and grammatical variants thereof, as used herein refers to an individual whose analyte level(s) are being measured or monitored. The computing devices described herein further allow improved user interaction therewith by allowing the user to access event information associated with the functioning of an analyte monitoring system that is communicably coupled thereto such that the user can self-troubleshoot and/or send such information to customer service personnel for assistance.

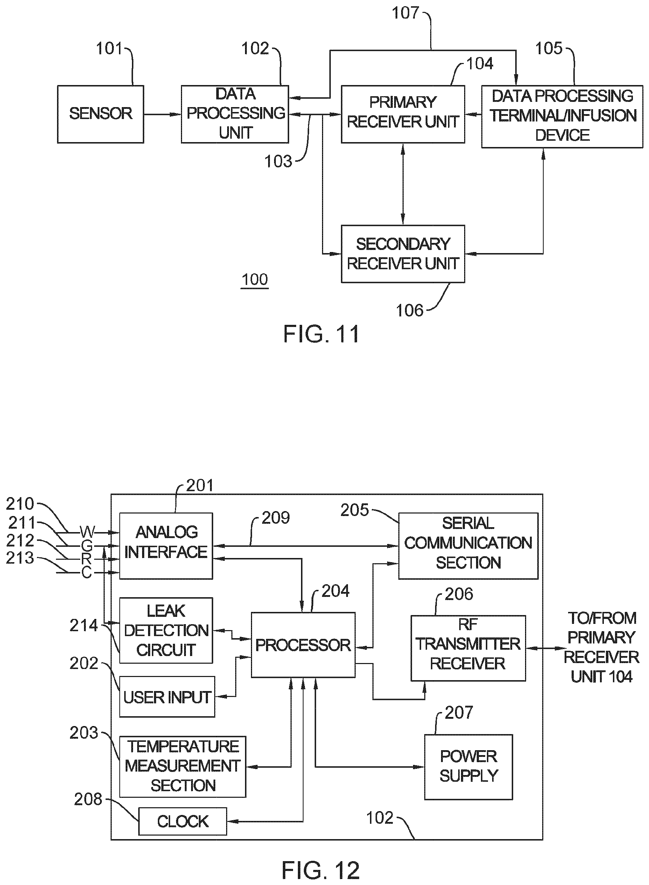

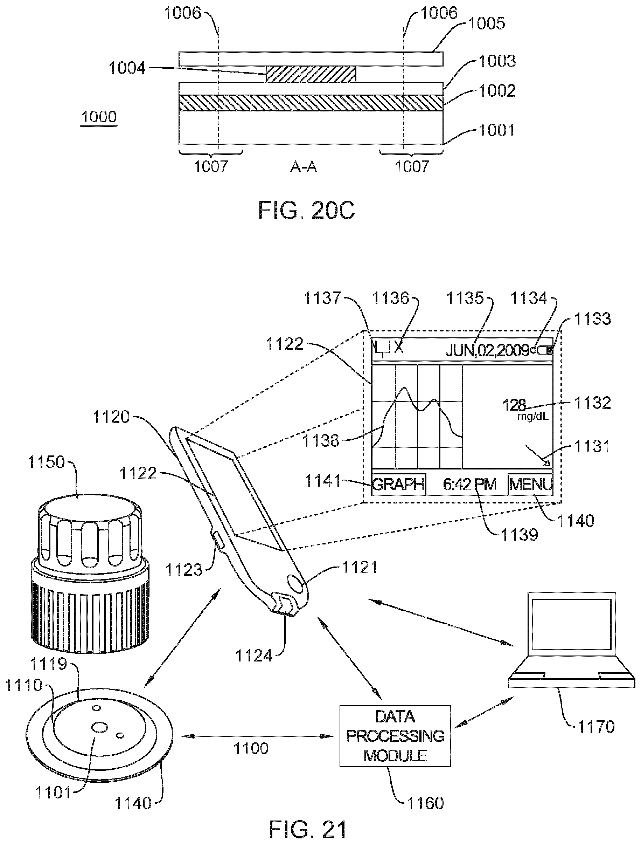

As used herein, the term "computing device," and grammatical variants thereof, refers to any kind of device that is capable of processing and displaying information including, but not limited to, a cell phone, a tablet, a receiver or data reader, a PDA, and the like, whether the display is in grayscale or color, and as further defined below with reference to display device 104, 106 (see FIG. 11) and 1120 (see FIG. 21). As used herein, the term "communicably coupled," and grammatical variants thereof, refers to any electronic communication between two components, whether wired or wireless and by any means, and includes components that are coupleable and not actively communicating.

In some embodiments, the computing devices of the instant application are preferably hand-held, such as a touchscreen cellular phone. As used herein, the term "lifestyle," and grammatical variants thereof, refers to a sensor user's behavioral patterns, including, but not limited to, food intake, activities, exercise, sleep patterns, stresses, and the like.

Often, computing devices associated with analyte monitoring sensors or other apparatuses for sending information to the computing device, are limited in their usability and require multiple steps for accessing data or activating particular functionalities. As used herein, the term "analyte monitoring sensor," "analyte sensor," or simply "sensor," and grammatical variants thereof, refers to any ex vivo or in vivo sensing device that can determine analyte levels of a body and transmit data therefrom associated with those analyte levels. In preferred embodiments, the analyte monitoring sensor is an in vivo sensor, such as a continuous analyte monitoring sensor. Sensors and sensing systems are described in greater detail herein below.

That is, conventional computing devices typically require data and functionality to be divided into multiple layers and views, requiring a user to scroll through many windows or regularly switch views, often wasting the time of the user. When such computing devices are coupled with analyte monitoring sensors designed to manage disease or monitor health, the inconvenience of these multiple layers and views can negatively influence the experience of the user, including discouraging use altogether.

Conventional computing devices associated with analyte monitoring sensors typically do not allow a user to input specific information about a sensor user's lifestyle that is easily enterable and thereafter easily accessible without the user having to navigate through many views. For example, conventional computing devices may layer various potential lifestyle inputs, without allowing customization or specific input of information, such that the date is limited (e.g., a meal without an associated carbohydrate amount, exercise without intensity or duration, and the like), and which is further not viewable in a single display window. However, linking a sensor user's lifestyle to a particular date and time of an analyte level measurement (e.g., concentration) may be critical to controlling a particular disease (e.g., diabetes) or to the health of the sensor user. The multiple steps characteristic of conventional computing devices can discourage a user from linking a sensor user's lifestyle events with their analyte levels, thereby potentially leading to poor management of a disease or adverse health consequences.

Additionally, the embodiments of the present disclosure allow a user quick access to events related to the functionality of a paired analyte monitoring sensor, thereby allowing the user to determine how to troubleshoot the operation of that sensor. Conventional computing devices do not provide this functionality, and thus may result in failure to obtain accurate analyte level measurements, thereby also resulting in poor management of a disease or adverse health consequences.

Accordingly, the embodiments described herein allow a user to access a snap-shot view of important sensor user lifestyle data and a snap-shot view important of events associated with the functionality of an analyte monitoring sensor. These snap-shot views bring together otherwise disparate data included in conventional computing devices, if included at all. The bringing together of such data encourages input of the lifestyle information and allows easy access to already summarized data associated with a sensor user's lifestyle (e.g., access by the sensor user, by a treating physician, by a loved one such as a parent or sibling, and the like). Accordingly, the embodiments of the present disclosure improve the performance of display screens and interactive interfaces associated with analyte sensor measurements, thereby improving the assessment and treatment various analyte-monitored diseases.

Access to such lifestyle snap-shots of information in relationship to particular analyte measurements (e.g., concentration at specific dates and times) permits quick and accurate adjustments to be made for the health of the user and/or determinations of positive or negative lifestyle choices. For example, a user may determine whether exercise is beneficial or detrimental to their analyte levels at a certain intensity and alter their exercise regimen accordingly. Users may pinpoint particular food groups that are beneficial or detrimental to their analyte levels and adjust dietary decisions accordingly.

Access to the event log snap-shots of information in relationship to particular analyte measurements (e.g., concentration at specific dates and times) of the present disclosure allows a user to troubleshoot potentially erroneous analyte measurements, which could lead to treatment actions that are unnecessary or potentially harmful (e.g., if a sensor is too cold for an accurate measurement, the user will know not to immediately inject insulin or consume certain foods to alter their glucose levels). Moreover, the combination of the sensor user's lifestyle information and the event log of the present disclosure will further permit a user to understand their analyte measurements more accurately to make appropriate treatment decisions.

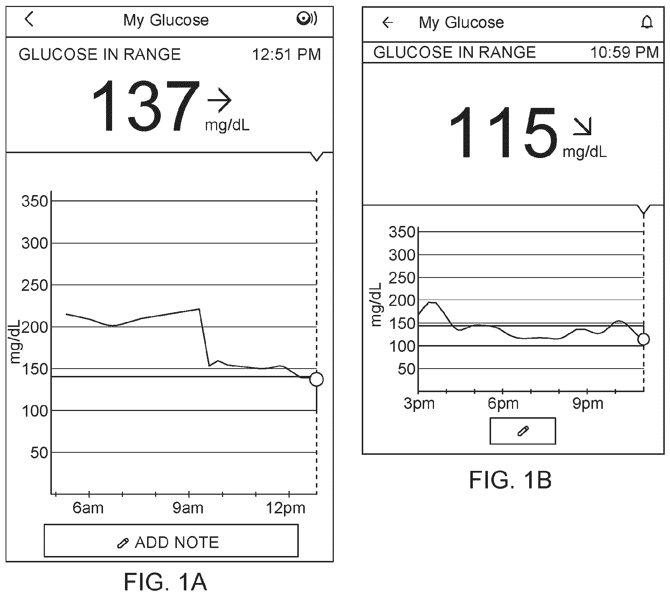

Although FIGS. 1A to 10B describe the computing devices of the present disclosure with reference to a glucose analyte monitoring sensor, it is to be understood that the computing devices of the present disclosure are suitable for use with any other type of analyte monitoring sensor (e.g., a lactate monitoring sensor), without departing from the scope of the present disclosure.

### Input of Information and Notes Corresponding to a Sensor User's Lifestyle

Referring now to FIGS. 1A and 1B, two exemplary embodiments of a display screen of a computing device are shown displaying an analyte monitoring scan display window, or more specifically in these embodiments, a "My Glucose" display window. As used herein, an "analyte monitoring scan display window" or simply "scan display window," and grammatical variants thereof, refers to a display window of a computing device having a display screen configured to show at least one characteristic of a measured analyte associated with a specific analyte scanning event (e.g., date and time), but which may include additional analyte measurements. The scan display window is distinguishable from an analyte monitoring daily display window of the computing device, discussed herein in detail below.

As shown in FIGS. 1A and 1B, the scan display window may include an icon in the upper right hand corner of the display window that when selected permits a user to scan an analyte monitoring sensor communicably coupled to the computing device having the display screen displaying the scan display window. For example, in one embodiment, actuating or selecting the "scan" icon (or any other selectable symbol or button that prompts scanning, e.g., "ready to scan" or "please scan," and the like) may automatically gather data from the computing devices described herein. In some embodiments, upon placing the computing device near an analyte monitoring sensor, a connection may be established (e.g., a near-field communication (NFC) connection) and data from the sensor may be transmitted to the computing device. In certain embodiments, the scan display window or other display window may automatically be activated to display to the use the sensor user's analyte levels (e.g., displaying a graphical representation, the actual analyte level or concentration, other derived analyte levels (e.g., A1c)), and the like. That is, one or more of display screens of the computing device may automatically alert a user (e.g., as a notification) of the scan data (e.g., the display window of FIGS. 1A or 1B may automatically appear on the computing device). The display may display any or all of the analyte level trend arrow, the trend analyte level message, the current analyte reading, and the like, as described below.

In some embodiments, if the computing device is idle *(e.g.,* a cellular phone in sleep mode), a notification banner may appear to alert a user *(e.g.,* a sensor user) to launch the scan display window and/or to scan the analyte monitoring sensor. That is, the computing device may be configured to prompt a user to scan a sensor user's analyte levels at particular times, which may be preconfigured or configured by the user, including the sensor user. For example, if the computing device is a cellular phone, even if the it is locked or otherwise is "sleep" mode (and any variation thereof), the scan display window will be displayed and/or another form of prompt will appear, without departing from the scope of the present disclosure. That is, if the computing device has a sleep mode, the embodiments described herein allow the computing device to communicate to a user the need to scan a sensor user's analyte levels, thereby allowing the user to easily obtain analyte level (e.g., glucose) without actually activating the computing device or removing it from sleep mode. In some embodiments, the computing device may, upon placing the computing device described herein (e.g., a cellular phone, tablet, PDA, fitness monitor or pedometer, and the like) near the analyte monitoring system, may automatically scan for analyte measurements. That is, physical scanning may or may not be required in the embodiments of the present disclosure.

Accordingly, the scan display window displays a specific past scan *(e.g.,* a scan having occurred immediately or shortly before the scan display window is displayed, showed as 137 mg/dL in FIG. 1A), but may further be used by the user to initiate a new scan (*e.g*., a back-to-back scan from the prior scan, or a subsequent scan after the elapse of any period of time). For example, a user may wish to perform a back-to-back scan immediately or shortly after the prior scan to test the accuracy of the coupled sensor.

The scan display window may further include one or more of a clock (digital or analog, as applicable throughout all display windows herein), the current analyte level concentration based on the last scan of the analyte monitoring sensor, a graphical representation of the analyte levels overtime, and a coded target range of analyte level (*e.g*., the shaded area between 100 and 140 mg/dL in FIGS. 1A and 1B). As shown in FIGS. 1A and 1B, the graphical representation of the analyte levels overtime is depicted with time on the x-axis and analyte level on the y-axis in milligrams per deciliter (mg/dL). Other units (*e.g*., use of a 24 hour-clock, use of millimoles per liter (mmol/L) for the analyte levels, and the like) or unit intervals may define the graphical representation, without departing from the scope of the present disclosure, provided that the analyte levels are linked to a specific date and time.

In some embodiments, as illustrated in FIGS. 1A and 1B, the scan display window may inform a sensor user whether their analyte level is in a particular target range by displaying an analyte level trend arrow and/or a trend analyte level message. The target range may be defined by the computing device or by a user (*e.g*., the sensor user), and accordingly may be adjustable in some embodiments to allow individualization.

Trend arrows may include a diagonal right arrow pointed upward to indicate that the analyte level is rising, a vertical arrow pointed up to indicate that the analyte level is quickly rising, a horizontal right or left (preferably right) arrow to indicate that the analyte level is steady or changing slowly, a diagonal arrow pointed downward to indicate that the analyte level is falling, and/or a vertical arrow pointed down to indicate that the analyte level is falling quickly. The trend analyte level messages may include language stating that the analyte level is above a high threshold, between target range and the high threshold, within target range, below a low threshold, or between target range and the low threshold. Alternatively, or in addition to the trend arrows and/or the trend messages, color-coding may be color-coded, such as orange, yellow, green, yellow, and red, respectively, to indicate that the analyte level is above a high threshold, between target range and the high threshold, within target range, below a low threshold, or between target range and the low threshold. In the embodiments of the present disclosure, trend arrows, trend messages, and color-coding may be displayed simultaneously or alternatively in the scan display window. Accordingly, one or multiple means of conveying the trend of a sensor user's analyte levels may be employed in order to suit a particular individual (e.g., a color-blind user may find the color-coding unhelpful and thus may rely on one or both of the trend arrows and/or messages). As shown in FIGS. 1A and 1B, the glucose analyte level is within the target range, showing each of a horizontal right trend arrow, a trend message of message of "GLUCOSE IN RANGE," and a color-coding of green.

Accordingly, the scan display window of the computing device(s) described herein having display screens may be is accessed upon scanning a sensor user's analyte level corresponding to the measurements provided by an analyte monitoring sensor. The scan display window may further be a display window through which other functionalities are accessed, including accessing the user input buttons associated with a sensor user's lifestyle at a date and time. As used herein, the term "button," and grammatical variants thereof, refers to an element of a computing device having a plurality of display screens that when actuated (e.g., pressed or contacted) causes some alteration in a particular display window, without limitation of size, style, texture, tactility, shape, and the like (e.g., embodied in a computer screen, hyperlink, keyboard, slide bar, scroll bar, and the like). It is to be understood that the various components of the scan display window, including, but not limited to, terminology, color-coding, arrow directions, scale, size, arrangement, and/or iconology may be varied, without departing from the scope of the present disclosure.

The scan display window of the present disclosure may include functionality to quickly access a limited number of user input buttons associated with a sensor user's lifestyle at a specific date and time. As depicted in FIGS. 1A and 1B, access to the user input buttons may be in the form of a button having an icon in the shape of a pen or pencil. In some embodiments, the pen or pencil icon may be depicted as pointing generally downward and toward the left, although other configurations are within the scope of the present disclosure. The icon may be alone or with accompanying text, such as "ADD NOTE" shown in FIG. 1A. As used herein, the term "add note button," and grammatical variants thereof, refers to a button as part of a display window (e.g., an scan display window) of a computing device having a plurality of display windows that allows user input about a sensor user's lifestyle, and is not limited to any particular terminology or iconology. For example, other text or symbols may be used alone or in combination without departing from the scope of the present disclosure, such as add diary, enter notes, a notepad icon, and the like.

The add note button may include an icon, and any accompanying text, that a user may select (e.g., via touchscreen in this embodiment) and transition to an input display window having the limited number of user input button associated with a sensor user's lifestyle at a specific date and time. It is to be understood that any other icon and/or text design or terminology that may prompt a user to understand that selecting an associated button therewith will result in access to an input display window may be used in accordance with the present disclosure, without departing from the scope herein.

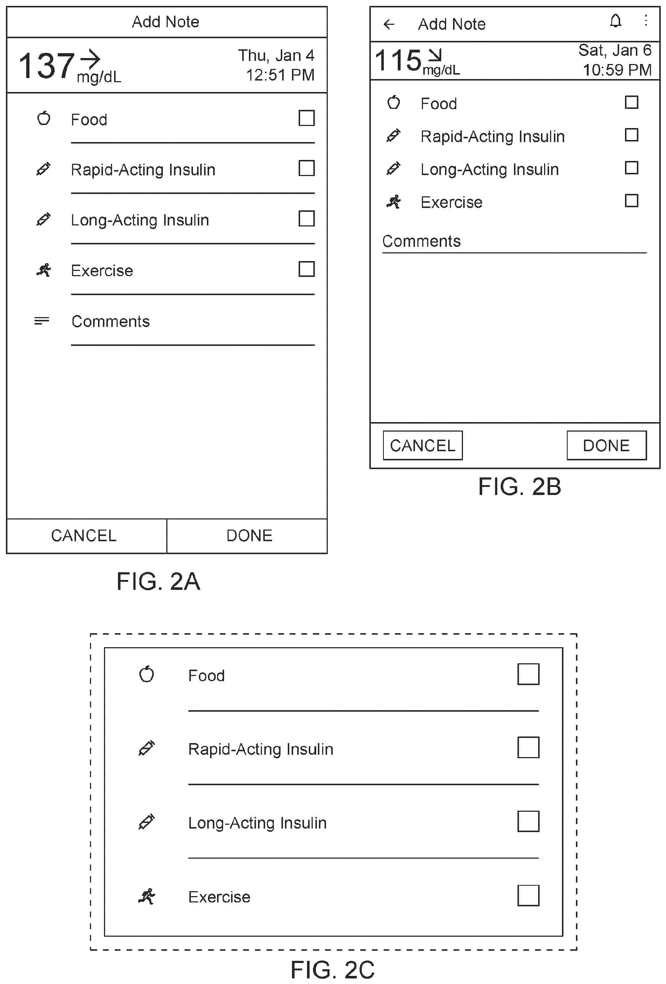

Accordingly, upon selecting the add note button (see FIGS. 1A and 1B), the computer device having the display screens of the present disclosure may transition to the input display window. Referring now to FIGS. 2A and 2B, two exemplary embodiments of a display screen of a computing device are shown displaying an input display window, or more specifically in these embodiments, an "Add Note" display window. As used herein, an "input display window," and grammatical variants thereof, refers to a display window of a computing device having a display screen configured to allow a user to input information about a sensor user's lifestyle, whether freeform or upon specific prompts.

The input display window of the present disclosure may include a listing of a limited number of user input buttons associated with a sensor user's lifestyle at a specific date and time. These user input buttons may be designed to track certain known influencers of an analyte being measured by an analyte monitoring sensor communicably coupled to the computing device. As shown in FIGS. 2A to 2C, such limited number of user input buttons may include, but are not limited to, Food, Rapid-Acting Insulin, Long-Acting Insulin, Exercise, Comments, and any combination thereof. The various user input buttons may be associated with various icons, as shown in FIGS. 2A and 2B. It is to be understood that such icons need not be present and that the particular styling of any present icons are not limited to those shown in FIGS. 2A and 2B, provided that they are representative of the particular user input button.

In some embodiments, the user inputs may be dynamic based on the information previously gleaned from the prior input information. For example, in some embodiments, the user input buttons that appear are associated with the most frequently used functions, like eating a meal or insulin bolus. In other embodiments, the computing device may be configured such that the display screens are predictive. For example, if a sensor user's analyte levels are high, the computing device may automatically display or offer a user input button to prompt the user (e.g., the sensor user) to input data related to the sensor user's lifestyle, such as a recent meal or insulin injection. That is, the computing device may be configured to detect certain spikes in analyte levels and prompt a user to input data that is related to the sensor user's lifestyle. For example, the Food user input button may appear if glucose has spiked because perhaps the sensor user just had a meal, or if glucose level suddenly drops, the Rapid- or Long- acting user input button may automatically appear because perhaps the sensor user was just administered an insulin bolus. Accordingly, a user may be prompt to enter an input based on the dynamic readings of an analyte monitoring sensor.

The user input buttons of the input display window may be selected by selecting the associated icon, the description of the user input button, and/or a selectable symbol (e.g., a check-box). For example, as shown in FIGS. 2A and 2B, the user input buttons of Food, Rapid-Acting Insulin, Long-Acting Insulin, and Exercise are selectable using a selectable symbol in the form of a check-box, whereas the input button of Comments is selectable upon selecting the word "Comments" or Comments icon (see FIG. 2A). Any variation of selectability is encompassed in the teaching of the present disclosure, as described herein, without departing from the scope of the thereof.

The input display window may further include a plurality of additional information for viewing or manipulation by the user of the computing device, including, but not limited to, the current analyte level concentration based on the last scan of the analyte monitoring sensor, a trend arrow and/or message, color-coding, the specific date and time, a selectable cancel button, and/or a selectable accept (or "DONE") button. Other features of the input display window may include a selectable scan button or icon, a selectable main menu button or icon, a selectable settings button or icon, and/or a selectable back button or icon, such as those shown in FIG. 2B, without departing from the scope of the present disclosure. It is to be understood that the various components of the input display window, including, but not limited to, terminology, color-coding, arrow directions, scale, size, arrangement, and/or iconology may be varied, without departing from the scope of the present disclosure, provided that a limited number of user input buttons is present for user input about a sensor user's lifestyle.

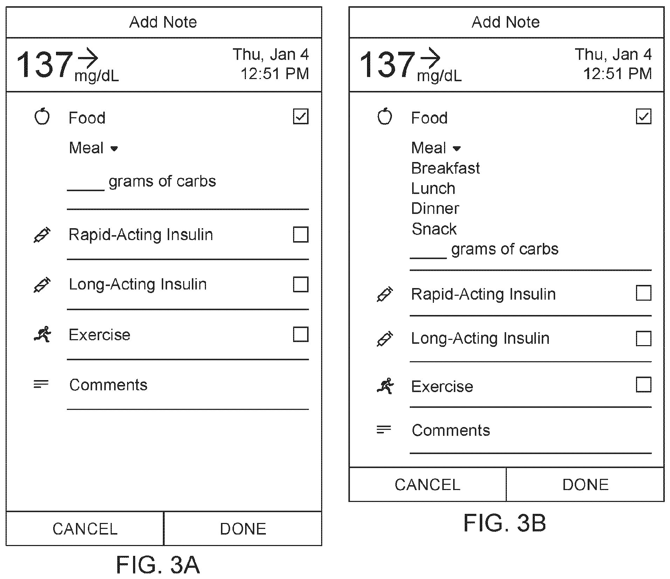

Referring now to FIGS. 3A to 3J, illustrated are a series of views of an input display window showing user interaction therewith, according to one or more embodiments of the present disclosure. Within the input display window (e.g., FIGS. 2A and 2B), a user can interact with the limited number of user input buttons displayed therein. When opting to input certain information about a sensor user's lifestyle using the limited number of user input buttons, any icons associated therewith may be highlighted or otherwise emphasized (e.g., by color, boldness, and the like) to illustrate to the user that the input has been made or is in process of being made. Each user input about a sensor user's lifestyle is linked via the electronics of the computing device, as described below, to the specific date and time at which the user input the information and accepted the input (e.g., selected the "DONE" button). In doing so, the user may track a sensor user's lifestyle choices in relation to particular analyte levels being measured or monitored by an analyte monitoring sensor. Moreover, as described below, the computing device having the display screen according to the embodiments described herein associates a sensor user's lifestyle information with analyte monitoring data directly on the scan display, and further allows direct access therefrom of the lifestyle information.

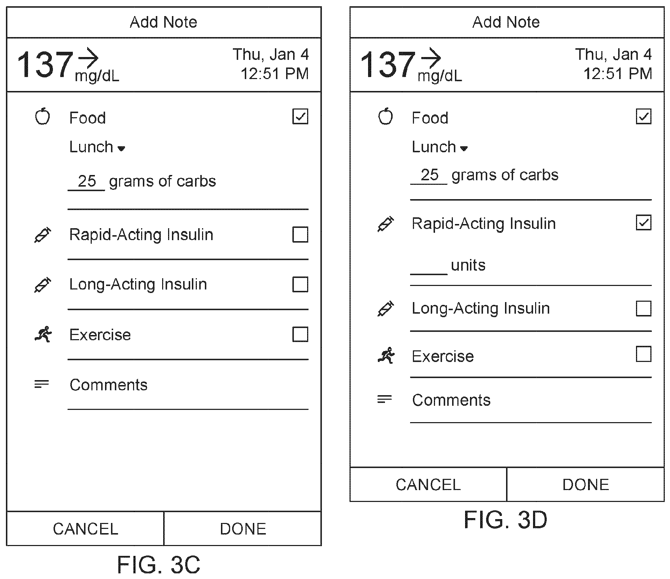

As shown in FIGS. 3A to 3C, a user may select the Food user input button by selecting the selectable symbol (e.g., the check-box) and additional information is thereafter prompted of the user within the input display window. In this embodiment, the user is prompted to select the appropriate meal for entry, which may be in the form of a drop-down menu, a scrolling menu, or other selectable menu type. The meal selections may include, but are not limited to, Breakfast, Lunch, Dinner, and Snack, without being bound to any particular order. As shown in FIG. 3C, upon selecting the appropriate meal (e.g., Lunch), the user may enter specific information about the meal, which may relate to the particular analyte levels being measured or monitored by an analyte monitoring sensor. As shown in FIG. 3C, the user may input the particular grams of carbohydrates associated with a sensor user's meal, which may be entered, for example, via a keyboard or touchscreen, via voice-activated text, and/or another enterable or selectable menu. Other specific information may also be prompted for entry by the user provided that it is associated with analyte levels of interest, such as specific types of sugars for glucose monitoring, without departing from the scope of the present disclosure.

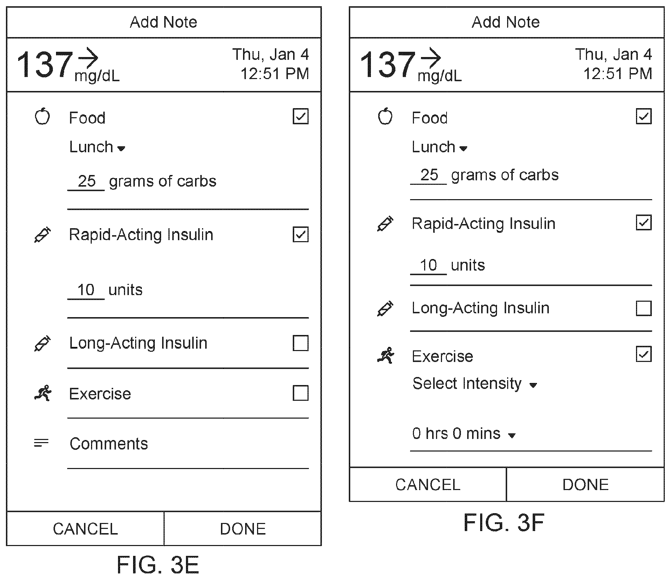

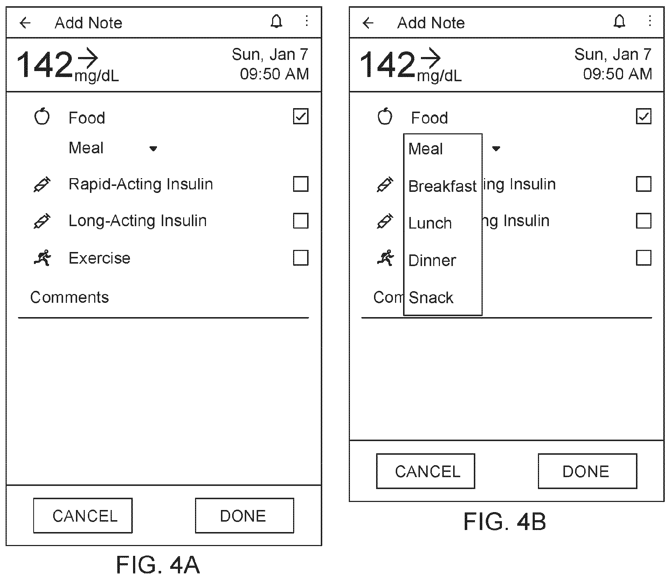

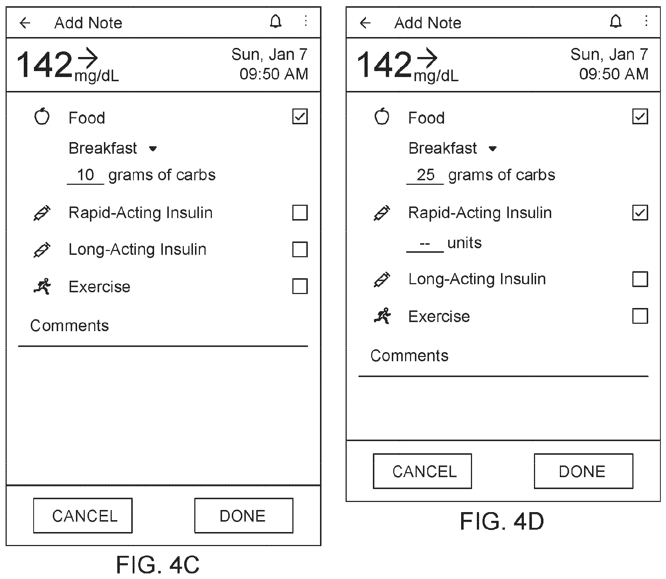

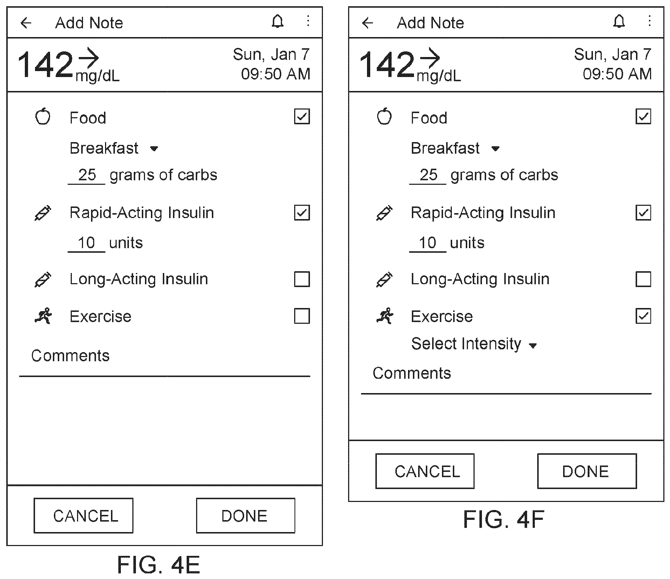

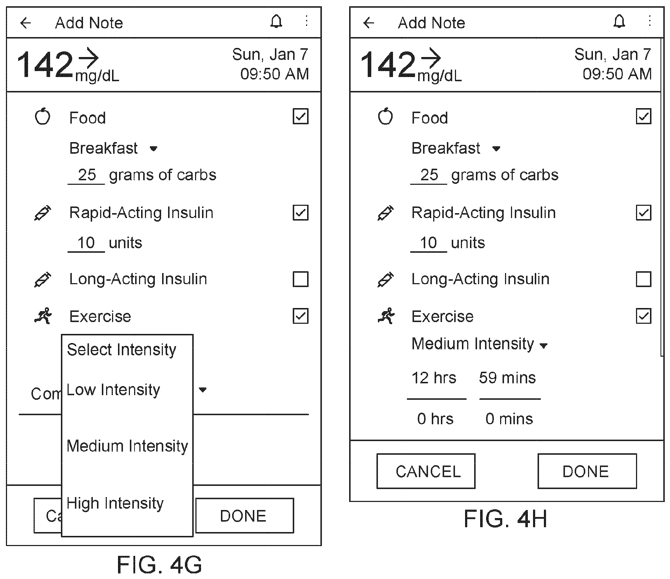

Upon entry of a single user input about a sensor user's lifestyle, the user may accept the entry and input into the input display window that the user has completed their entry (e.g., by selecting the DONE button). Alternatively, the user may wish to continue to input additional information about a sensor user's lifestyle. FIGS. 3D and 3E depict the user having already entered the Food input further selecting the selectable symbol for entry of Rapid-Acting Insulin, where thereafter the user may be prompted to enter the specific units of the rapid-acting insulin taken by the user at that particular date and time. Although not shown, the user may similarly select the selectable symbol for entry of a dosage of Long-Acting Insulin (e.g., in units). As shown in FIG. 3E, upon entry of additional input about a sensor user's lifestyle, any prior entries remain visible and editable to the user to ensure that a full picture of a sensor user's lifestyle at that specific date and time is accurately captured. If the user has input multiple entries of information about a sensor user's lifestyle, the input display window may include a scroll bar (*e.g*., to the right or left of the display window) to allow the user to access information that exceeds the size of the display screen of the computing device (see FIGS. 4H to 4J showing a scroll bar on the right side of the display window).

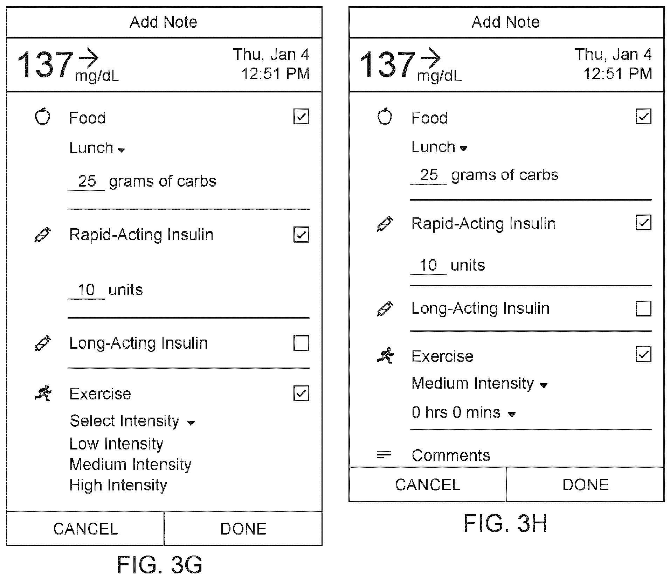

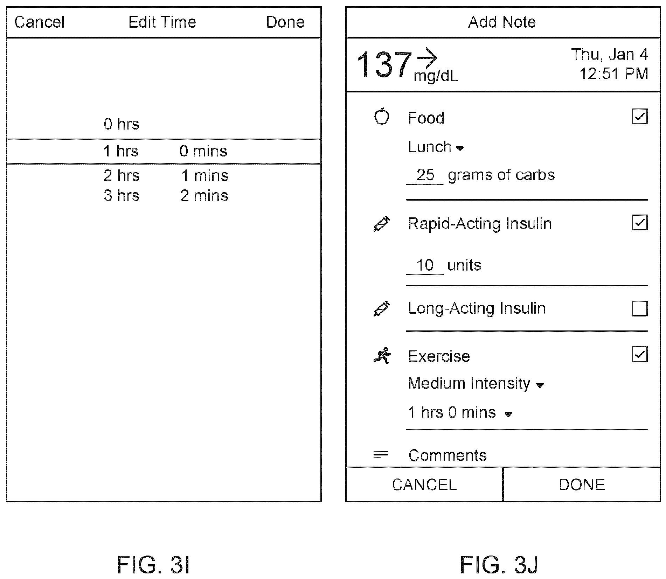

FIGS. 3F and 3J depict the user further selecting the selectable symbol for entry of Exercise, where thereafter the user may be prompted select a specific energy intensity level. For example, the "Select Intensity" prompt shown in FIG. 3F may provide for a selectable menu (e.g., drop-down menu, scrollable menu, and the like) allowing the user to select a specific intensity, such as the options shown in FIG. 3G of Low Intensity, Medium Intensity, and High Intensity. Upon selection of the specific exercise intensity by the user, and as shown in FIG. 3H, the user may be prompted to enter in the duration of the exercise. As shown in FIGS. 3H, a selectable menu for entering duration may be selected by the user, upon which the display screen of the computing device may transition to a time duration display window (see FIG. 3I).

As used herein, a "time duration display window," and grammatical variants thereof, refers to a display window of a computing device having a display screen configured to allow a user to select or input a particular time duration. As shown in FIG. 3I, the time duration display window may include a selectable menu for entering hour and minute duration information, depicted as a scrolling menu in FIG. 3I, but which may be any form of selectable menu, including allowing a user to enter in (via typing, text, or voice activated entry, and the like) hour and minute duration information. In some embodiments, the time duration display window further permits entry of other time intervals, such as seconds, without departing from the scope of the present disclosure. The time duration display may further include other features and functionalities, without departing from the scope of the present disclosure, such as a title of the time duration display window (e.g., "Edit Time"), a selectable cancel button, and/or a selectable accept (or "Done") button. Upon accepting the entered time, the display screen of the computing device transitions back to the input display window.

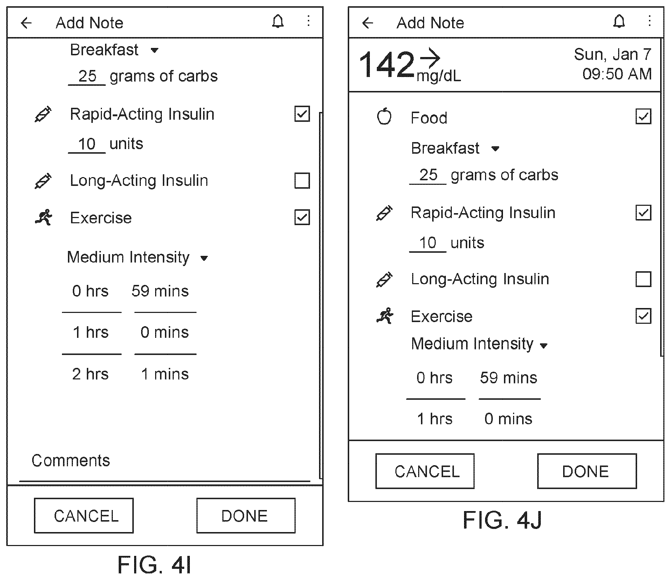

In other embodiments, upon the user selecting the selectable symbol for entry of Exercise and thereafter selecting a specific energy intensity level, rather than transitioning to the time duration display window, the selectable menu for entering hour and minute duration information may appear directly on the input display window (see FIGS. 4H and 4I). In such embodiments, the information is directly input into the input display window and viewable with the additional input information that the user input related to a sensor user's lifestyle.

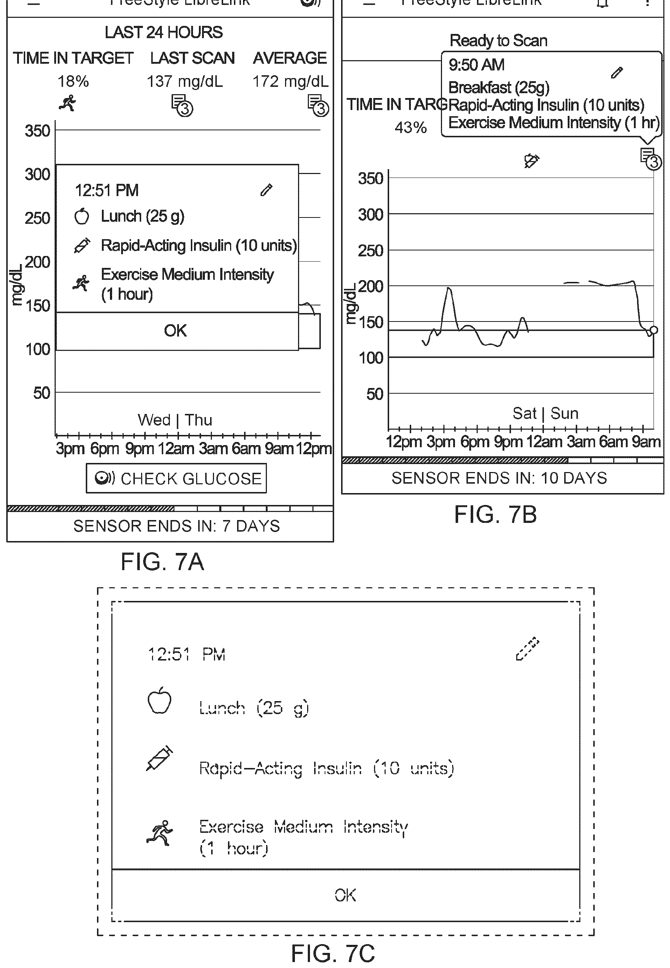

Although not shown, a user may additionally input comments into the input display window, which may be via a keyboard or touchscreen, via voice-activated text, or a selectable menu having specific pre-coded narratives. These pre-coded narratives may be included as part of the computing device or may be configurable by the user. For example, such narratives may be related to stress, sleep patterns, or other common lifestyle events associated with the life of the sensor user. When included, these comments may be, but need not be, visible along with the other input information in the input display screen, without departing from the scope of the present disclosure (as well as in the pop-up display window of FIGS. 7A and 7B).

FIGS. 4A to 4J illustrate a series of views of the input display window according to one or more embodiments described herein showing user interaction therewith, according to one or more embodiments of the present disclosure. FIGS. 4A to 4J represent embodiments that are different in aesthetics and certain features, but are substantially similar to the embodiments described above with reference to FIGS. 3A to 3J and, accordingly, will not be again discussed in detail herein.

FIGS. 3J and 4J represent a user's completed input display screen, according to one or more embodiments of the present disclosure, allowing the user to view all input information in a single location and to accept the entry information (e.g., by selecting the "DONE" button). It is to be understood that any or all of the user input buttons may have been selected and information input about a sensor user's lifestyle, without departing from the scope of the present disclosure, including Comments input.

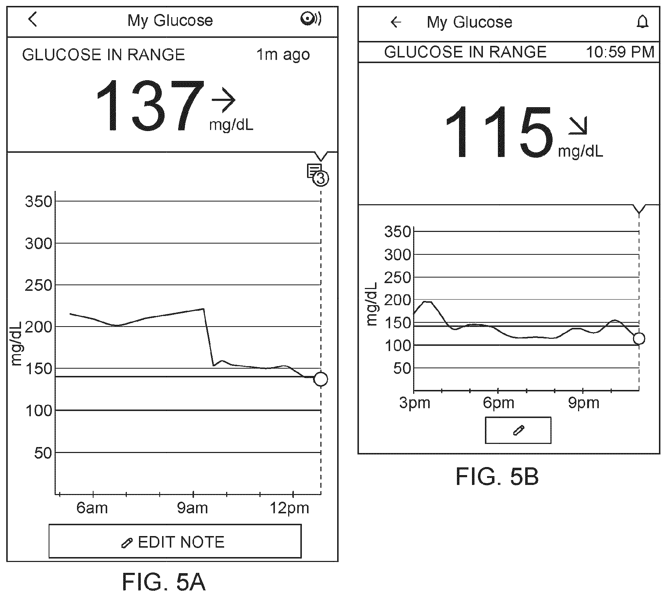

Upon accepting the input information associated with a sensor user's lifestyle, the display screen of the computing device may transition again to the scan display window and associate the particular input data with the specific date and time that the inputs were accepted, which may display the particular inputs as a selectable icon (see FIG. 5A). As shown in FIGS. 5A and 5B, the scan display window may be updated to display the time at which the input information was accepted by the user and associate such a time with a particular analyte level. Alternatively, the user input information may be automatically associated with the specific date and time of the last scan if the data is input within a finite duration of time after the scan (e.g., less than 3 or 5 minutes), or the user may input a particular date and time for association, without departing from the scope of the present disclosure.

Visually, the time may be viewed as a clock or as an amount of time that has elapsed since the last scan and/or user input. The scan display window may display the last scan as a hatched line in a graphical representation of analyte levels over a relatively short period of time (e.g., 8 to 12 hours), may include a selectable icon or other selectable symbol to indicate that user information is associated with the particular scan or analyte level at the particular time, and/or include an selectable edit button to allow a user to input additional notes and/or edit the notes already input (e.g., "EDIT NOTE" of FIG. 5A or pencil or pen icon of FIG. 5B). As shown, an icon or other symbol may be used to indicate that user information has been input for a particular date and time and may be editable by selecting an selectable edit button or by selecting an icon or symbol directly, without departing from the scope of the present disclosure.

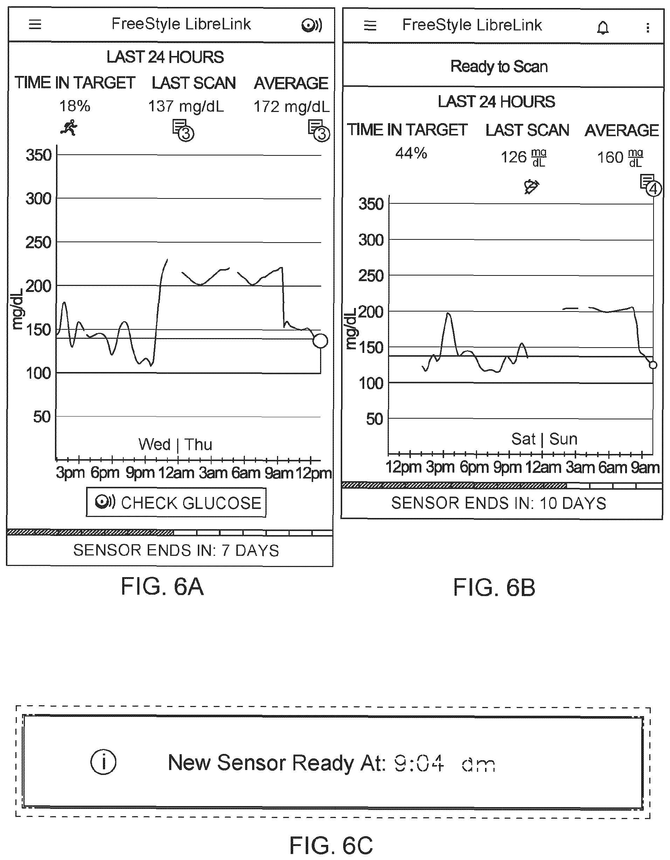

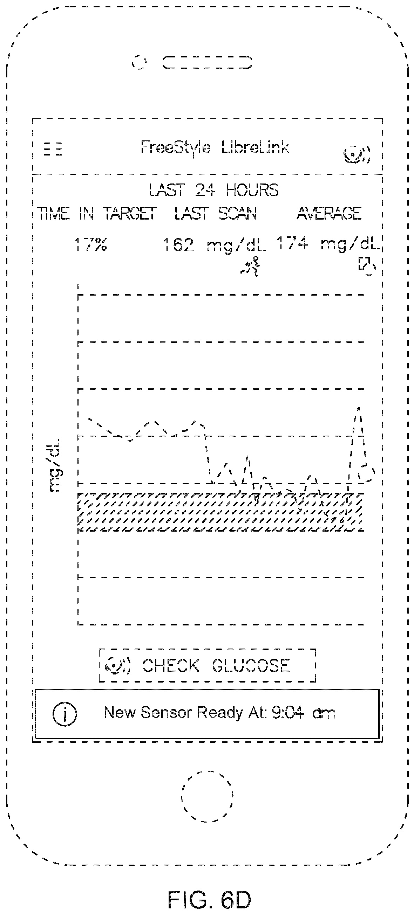

An analyte monitoring daily display window of the computing device may be accessed by transitioning from the analyte monitoring scan display window, such as hitting the back arrow icon shown in the upper left corner of FIGS. 5A and 5B, or other manner of transitioning the display windows. As used herein, the term "analyte monitoring daily display window" or simply "daily display window," and grammatical variants thereof, refers to a display window of a computing device having a display screen configured to show a plurality of measured analyte levels (e.g., concentration), each associated with a specific date and time and over a period of at least 24 hours. The daily display window may be the primary display window of the computing devices described herein. Representative embodiments of daily display windows in accordance with one or more embodiments of the present disclosure are shown in FIGS. 6A to 6D.

As shown in FIGS. 6A to 6D, features of the daily display window may include, but are not limited to, an icon banner indicating a countdown of sensor life for an associated analyte monitoring sensor (e.g., in days and hours, represented by color-changing or shape-changing graphics, such as bars), a graphical representation of the analyte levels over a time-period of at least 24 hours, a coded target range of analyte level (e.g., the shaded area between 100 and 140 mg/dL in FIGS. 6A and 6B), a selectable scan button or icon (e.g., upper right icon in FIG. 6A or bell icon in FIG. 6B), a selectable main menu button or icon, (e.g., hamburger icon in upper left corner of FIGS. 6A and 6B), a selectable settings button or icon (e.g., vertical dot icon in upper right corner of FIG. 6B), an indication of the time period represented by the daily display window (e.g., "Last 24 Hours"), an indication of when a new sensor is ready to be used (e.g., an indication of its warm-up time remaining or of the time that the sensor will be ready) including an icon ("i") indicating that such information is being displayed, and/or various data related to the analyte levels during the period of measurement (e.g., "TIME IN TARGET," "LAST SCAN," "AVERAGE," and the like). In some embodiments, the selectable settings button or icon may be consolidated, such that information regarding such settings, and described below, are located within the selectable main menu *(i.e.,* rather than having two separate menus).

In addition to these features, and as shown in FIGS. 6A and 6B, the daily display window may display one or more selectable symbols correlating to the input data by a user about the a sensor user's lifestyle described above. The selectable symbols may be positioned along the timeline of the graphical representation, such that their location is correlative to the date and time that the particular input was recorded. In so doing, the input information about a sensor user's lifestyle may be correlated to a particular analyte level, thereby allowing a sensor user to make informed decisions about future lifestyle choices and their effect on particular analyte levels. As shown in FIGS. 6A and 6B, the date may be a relative showing with reference to the current date (e.g., Wed/Thu in FIG. 6A and Sat/Sun in FIG. 6B) and/or the actual date may be displayed. Other features may be displayed on the daily display window of the computing devices described herein, without departing from the scope of the present disclosure. It is further to be understood that the various components of the daily display window, including, but not limited to, terminology, color-coding, arrow directions, scale, size, arrangement, and/or iconology may be varied, without departing from the scope of the present disclosure.

The selectable symbols (or icons) of the daily display window may be any signals indicative of the summary of information that was input by the user. In some embodiments, the selectable symbols of the daily display window may be single symbols (e.g., the running person symbol in FIG. 6A), two or more overlaid symbols (e.g., the apple and syringe symbols in FIG. 6B), or a stacked symbol showing a number representing the number of inputs for the specific date and time (e.g., the stacked symbols showing the number "3" in FIG. 6A and the number "4" in FIG. 6B). Any other symbols may be suitable, without departing from the scope of the present disclosure, provided that they are representative of the user input information, and may or may not correlate to the symbols displayed (if at all) in the input display window.

A user may select one of the selectable icons from the daily display window to display a pop-up display window of the summary of the input information overlaid upon the daily display window, as shown in the embodiments of FIGS. 7A to 7C. As shown, the pop-up display window may include the time of entry and a summary of the input information input by the user, which may vary depending on what limited user input buttons the user chose to select and provide input *(e.g.,* see FIGS. 2A and 2B above). The pop-up display window may include any summary that is indicative of the information input by the user including, but not limited to, the associated icon, the description of the user input button, and the input date provided by the user, as shown in FIGS. 7A to 7C. Additionally, the pop-up display window may include a selectable edit icon (e.g., the pencil or pen icon, or any other form of a selectable edit button) located at a location within the pop-up display window that is selectable to allow the user to again access the input display window and alter their inputs, for example, should such alteration be necessary to ensure the accuracy of the input. Further, in some embodiments, a selectable accept button (e.g., "OK") may be included, wherein upon selection of the accept button, the pop-up display window is closed (e.g., undisplayed or no longer displayed) to again reveal the daily display window in its entirety. Alternatively, or additionally, a user may select a portion of the pop-up display window *(i.e.,* not a selectable edit icon button or accept button) to undisplay the pop-up display window and again reveal the daily display window in its entirety, or a user may select a portion of the daily display window (i.e., not an otherwise selectable button) to undisplay the pop-up display window and again reveal the daily display window in its entirety.

Other features may be displayed on the pop-up display window of the computing devices described herein, without departing from the scope of the present disclosure, provided that it contains a summary of the input information at the particular date and time related to a sensor user's lifestyle. It is further to be understood that the various components of the daily display window, including, but not limited to, terminology, color-coding, arrow directions, scale, size, arrangement, and/or iconology may be varied, without departing from the scope of the present disclosure.

### Event Log Associated with Analyte Monitoring Sensor

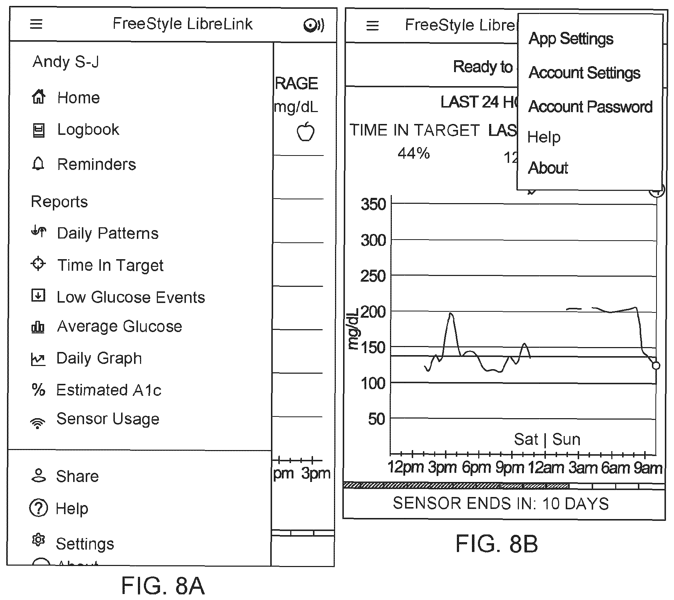

As described above, the computing devices comprising the plurality of display screens of the present disclosure may comprise an event log associated with an analyte monitoring sensor at a specific date and time. Accordingly, the computing device can track the functioning of the analyte monitoring sensor, allow a user to access the event log of the analyte monitoring sensor for monitoring or troubleshooting, as well as permitting a user to transmit the event log data to a customer service representative that is able to assist the user in troubleshooting the sensor. FIGS. 8A to 10B illustrate one or more embodiments of the computing devices described herein allowing a user to access and transmit an event log of a communicably coupled analyte monitoring sensor. It is to be understood that the various features of FIGS. 8A to 10B, including, but not limited to, terminology, color-coding, scale, size, arrangement, and/or iconology may be varied, without departing from the scope of the present disclosure.

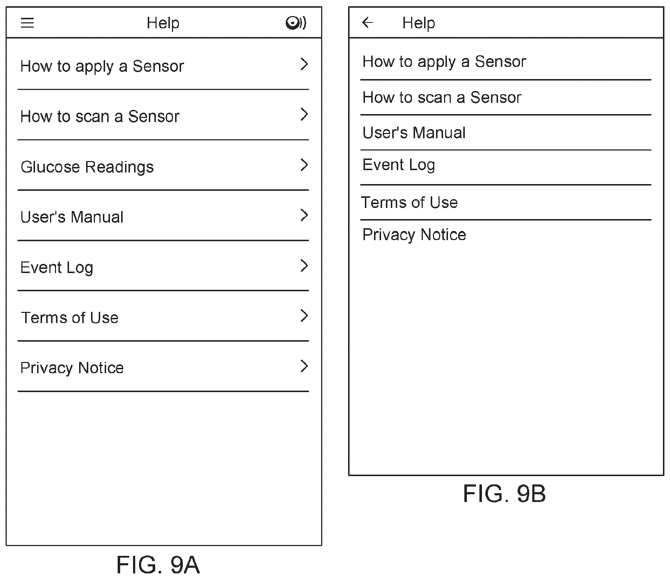

Referring now to FIGS. 8A and 8B, illustrated are display screens of the computing devices of the present disclosure displaying various user selectable buttons accessible from a selectable main menu button or icon or a selectable settings button or icon, according to one or more embodiments of the present disclosure. The user selectable buttons may be generalized buttons for navigating the plurality of display screens of the computing device, which in some embodiments may be accessed via an icon or menu symbol (e.g., a hamburger icon or vertical dot icon). The generalized user selectable buttons, accordingly, allow user selection for accessing various display screens associated with the computing device and/or an analyte monitoring sensor communicably coupled thereto. Any suitable user selectable button may be included in the embodiments shown in FIGS. 9A and 9B, without departing from the scope of the present disclosure, including, but not limited to, a Home display window, a Logbook display window, a Reminders display window, a Reports display window associated with various patterns of use (e.g., Daily Patterns, Time In Target, Low or High Analyte *(e.g.,* Glucose) Events, Average Analyte *(e.g.,* Glucose) levels, Daily Graphs, Estimated analyte or analyte-associated levels (e.g., A1c), and/or Sensor Usage), a Settings display window, a Share display window, an About display window, an Account display window, and/or a Help display window. Any one or more icons may or may not be associated with the user selectable buttons, without limitation.

Upon selecting one of the generalized user selectable buttons from a main menu or settings menu (collectively referred to herein as a "main menu"), a user may be directed to a new menu display window showing a listing of a limited number of additional user selectable buttons including an event log button. As shown in FIGS. 9A and 9B, the generalized button may be a "Help" button, which transitions to the menu display window having the limited number of user selectable buttons including the Event Log button. In the non-limited embodiments shown in FIGS. 9A and 9B, other user selectable buttons displayed on the menu display window may include, but are not limited to, How to apply a Sensor, How to scan a Sensor, Analyte (e.g., glucose) Readings, User's Manual, Terms of Use, and/or Privacy Notice. It is to be understood that while the event log button is depicted as part of a Help menu display window in FIGS. 9A and 9B, the location of the event log button may be accessible via any other of the generalized user selectable buttons described above, without departing from the scope of the present disclosure. The menu display window (as shown as the Help menu display window in FIGS. 9A and 9B) may further include a selectable scan button or icon, a main menu or settings menu icon, and/or a back button, among other potential features.

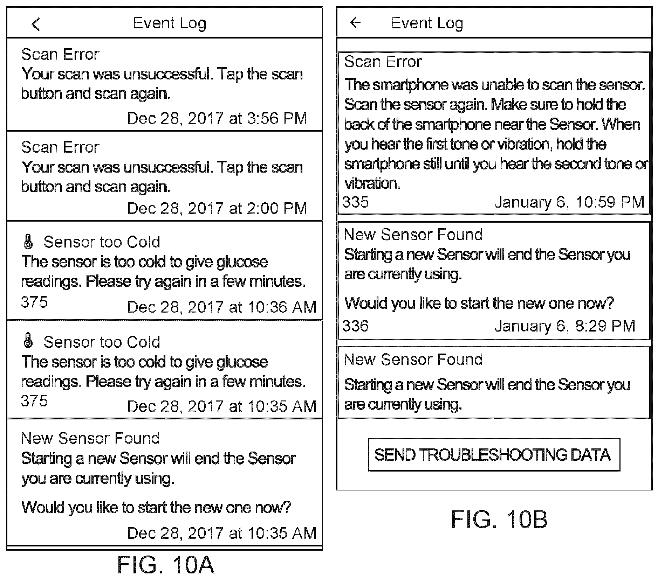

A user may select the event log button and be directed to the event log of the computing device of the present disclosure. That is, upon a user selecting the event log button, the computing device transitions to an event log display window. As used herein, the term "event log display window," and grammatical variants thereof, refers to a display window of a computing device having a display screen configured to show at least one event associated with an analyte monitoring sensor at a specific date and time. FIGS. 10A and 10B demonstrate embodiments of an event log display window, according to one or more embodiments of the preset disclosure. As shown, each event may, but need not, be accompanied by an event association number (e.g., "375" in FIG. 10A and "335" and "336" in FIG. 10B), an event title, an event description, an event icon or symbol, and/or the date and time that the event occurred, among other potential features.

In some embodiments, the event log logs events related to errors in scanning the analyte monitoring sensor, events related to the temperature of the sensor (e.g., the sensor may be too cold to accurately provide analyte measurements), and/or the sensing of a new sensor. Any suitable events associated with the functioning of the sensor may additionally be included in the event log, without departing from the scope of the present disclosure. In some embodiments, the event log prompts the user and/or sensor user to take a particular action, such as starting analyte measurement or monitoring using a new sensor that was sensed by the computing device. In other embodiments, the event log may further display a link or a page number of a user manual that describes the event (e.g., which may be an error event) and the associated remediation steps. The link may be a link to a user manual stored on the device or a website containing information about the error. If the computing device receives multiple event log entries, the event log display window may include a scroll bar (e.g., to the right or left of the window) to allow the user to access information that exceeds the size of the display screen of the computing device, as shown in FIGS. 10A and 10B.

While the event log may be useful to a user of the computing device and associated sensor, the event log display window may further allow the user to send the event log data to customer service personnel, such as the manufacture of the sensor experiencing the events. The event log data may be transmitted to customer service personnel using a user selectable button, such as a "SEND TROUBLESHOOTING DATA BUTTON," as shown in FIG. 10B. As used herein, the term "send troubleshooting data button," and grammatical variants thereof, refers to a user selectable button that is able to transmit event log information associated with an analyte monitoring sensor, regardless of the terminology, size, shape, etc. of the specific button. Alternatively, or additionally, the send trouble shooting data button may transmit data to customer service personnel associated with the computing device as well as the manufacturer of the sensor. In other embodiments, upon receipt of the data event log, an acknowledgement of receipt message may be sent back to the user in form of a banner, icon, or other symbol. The message may contain further information on remediation measures that may be taken by the customer service personnel, such as alerting the user that sensor is malfunctioned, advising the user to stop using the sensor, alerting the user that a new replacement sensor is being sent, or combinations thereof.

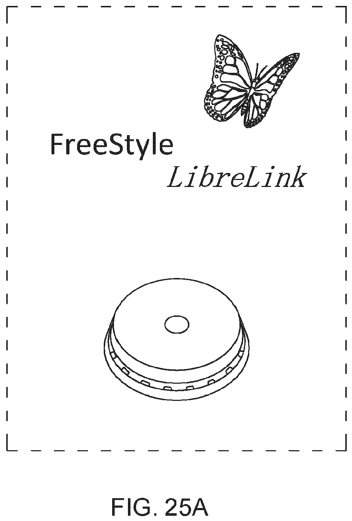

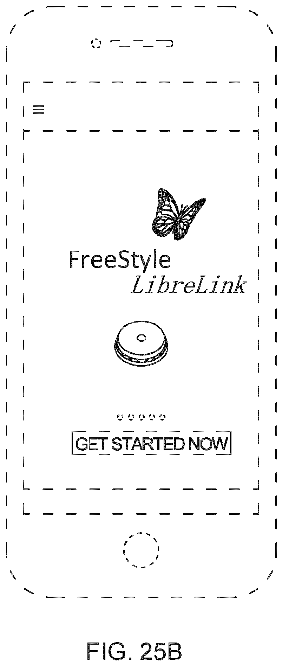

Referring now to FIGS. 25A and 25B, various display screens of a computing device presenting a start-up display window that are compatible with one or more embodiments of the present disclosure are shown. The start-up display window may include various elements, including, as shown, a brand name (e.g., FreeStyle^{™} LibreLink^{™}), the analyte monitoring device that may be communicably coupled to the computing device (e.g., a glucose sensor), one or more brand icons (e.g., a butterfly), a button allowing access into a plurality of additional display screens, selectable main menu button or icon, and/or a selectable settings button or icon.

### Example Embodiments of In Vivo Analyte Monitoring Systems

Referring now to FIG. 11, the analyte monitoring system 100 includes an analyte monitoring sensor 101, a data processing unit 102 connectable to the sensor 101, and a primary receiver unit or display device 104. In some instances, the primary display device 104 is configured to communicate with the data processing unit 102 via a communication link 103. In some embodiments, the primary display device 104 may be further configured to transmit data to a data processing terminal 105 to evaluate or otherwise process or format data received by the primary display device 104. The data processing terminal 105 may be configured to receive data directly from the data processing unit 102 via a communication link 107, which may optionally be configured for bi-directional communication. Further, the data processing unit 102 may include electronics and a transmitter or a transceiver to transmit and/or receive data to and/or from the primary display device 104 and/or the data processing terminal 105 and/or optionally a secondary receiver unit or display device 106.

Also shown in FIG. 11 is an optional secondary display device 106, which is operatively coupled to the communication link 103 and configured to receive data transmitted from the data processing unit 102. The secondary display device 106 may be configured to communicate with the primary display device 104, as well as the data processing terminal 105. In some embodiments, the secondary display device 106 may be configured for bi-directional wireless communication with each of the primary display device 104 and the data processing terminal 105. As discussed in further detail below, in some instances, the secondary display device 106 may be a de-featured receiver as compared to the primary display device 104, for instance, the secondary display device 106 may include a limited or minimal number of functions and features as compared with the primary display device 104. As such, the secondary display device 106 may include a smaller (in one or more, including all, dimensions), compact housing or be embodied in a device, such as a wrist watch, arm band, PDA, mp3 player, a cellular phone, and the like, for example. Alternatively, the secondary display device 106 may be configured with the same or substantially similar functions and features as the primary display device 104. The secondary display device 106 may include a docking portion configured to mate with a docking cradle unit for placement by, for example, the bedside for nighttime monitoring, and/or a bi-directional communication device. A docking cradle may recharge a power supply.

The computing devices having the plurality of display screens described herein may be either or both of the primary display device 104 and/or the secondary display device 106, or display device 1120, in accordance with the embodiments of the present disclosure.

Only one analyte sensor 101, data processing unit 102, and data processing terminal 105 are shown in the embodiment of the analyte monitoring system 100 illustrated in FIG. 11. However, it will be appreciated by one of ordinary skill in the art that the analyte monitoring system 100 may include more than one sensor 101 and/or more than one data processing unit 102, and/or more than one data processing terminal 105. Multiple sensors may be positioned in a user for analyte monitoring at the same or different times. In some embodiments, analyte information obtained by a first sensor positioned in a user may be employed as a comparison to analyte information obtained by a second sensor. This may be useful to confirm or validate analyte information obtained from one or both of the sensors. Such redundancy may be useful if analyte information is contemplated in critical therapy-related decisions. In some embodiments, a first sensor may be used to calibrate a second sensor.

In a multi-component environment, each component may be configured to be uniquely identified by one or more of the other components in the system so that communication conflict may be readily resolved between the various components within the analyte monitoring system 100. For example, unique IDs, communication channels, and the like, may be used.

In some embodiments, the sensor 101 is physically positioned in or on the body of a user whose analyte level is being monitored. The sensor 101 may be configured to at least periodically sample the analyte level of the user and convert the sampled analyte level into a corresponding signal for transmission by the data processing unit 102. The data processing unit 102 is coupleable to the sensor 101 so that both devices are positioned in or on the user's body, with at least a portion of the analyte sensor 101 positioned transcutaneously. The data processing unit 102 may include a fixation element, such as an adhesive or the like, to secure it to the user's body. A mount (not shown) attachable to the user and mateable with the data processing unit 102 may be used. For example, a mount may include an adhesive surface. The data processing unit 102 performs data processing functions, where such functions may include, but are not limited to, filtering and encoding of data signals, each of which corresponds to a sampled analyte level of the user, for transmission to the primary display device 104 via the communication link 103. In some embodiments, the sensor 101 or the data processing unit 102 or a combined sensor/data processing unit may be wholly implantable under the skin surface of the user.

In some embodiments, the primary display device 104 may include an analog interface section including an RF receiver and an antenna that is configured to communicate with the data processing unit 102 via the communication link 103, and a data processing section for processing the received data from the data processing unit 102 including data decoding, error detection and correction, data clock generation, data bit recovery, etc., or any combination thereof.

In operation, the primary display device 104 in some embodiments is configured to synchronize with the data processing unit 102 to uniquely identify the data processing unit 102, based on, for example, an identification information of the data processing unit 102, and thereafter, to periodically receive signals transmitted from the data processing unit 102 associated with the analyte levels monitored by the sensor 101.

With continued reference to FIG. 11, the data processing terminal 105 may include a personal computer, a portable computer including a laptop or a handheld device (e.g., a personal digital assistant (PDA), a telephone including a cellular phone (e.g., a multimedia and Internet-enabled mobile phone including an iPhone^{®}, a Blackberry^{®}, an Android phone, or similar phone), an mp3 player *(e.g.,* an iPOD^{TM}, etc.), a pager, and the like), and/or a drug delivery device *(e.g.,* an infusion device), each of which may be configured for data communication with the display devices via a wired or a wireless connection. Additionally, the data processing terminal 105 may further be connected to a data network (not shown) for storing, retrieving, updating, and/or analyzing data corresponding to the detected analyte level of the user.

The data processing terminal 105 may include a drug delivery device (e.g., an infusion device) such as an insulin infusion pump or the like, which may be configured to administer a drug (e.g., insulin) to the user, and which may be configured to communicate with the primary display device 104 for receiving, among other things, the measured analyte level. Alternatively, the primary display device 104 may be configured to integrate an infusion device therein so that the primary display device 104 is configured to administer an appropriate drug (e.g., insulin) to users, for example, for administering and modifying basal profiles, as well as for determining appropriate boluses for administration based on, among others, the detected analyte levels received from the data processing unit 102. An infusion device may be an external device or an internal device, such as a device wholly implantable in a user.

In some embodiments, the data processing terminal 105, which may include an infusion device, such as an insulin pump, may be configured to receive the analyte signals from the data processing unit 102, and thus, incorporate the functions of the primary display device 104 including data processing for managing the user's insulin therapy and analyte monitoring. In some embodiments, the communication link 103, as well as one or more of the other communication interfaces shown in FIG. 11, may use one or more wireless communication protocols, such as, but not limited to: an RF communication protocol, an infrared communication protocol, a Bluetooth enabled communication protocol, an 802.11x wireless communication protocol, or an equivalent wireless communication protocol which would allow secure, wireless communication of several units (for example, per Health Insurance Portability and Accountability Act (HIPPA) requirements), while avoiding potential data collision and interference.

FIG. 12 is a block diagram depicting an embodiment of a data processing unit 102 of the analyte monitoring system shown in FIG. 11. User input and/or interface components may be included or a data processing unit may be free of user input and/or interface components. In some embodiments, one or more application-specific integrated circuits (ASIC) (e.g., having processing circuitry and non-transitory memory for storing software instructions for execution by the processing circuitry) may be used to implement one or more functions or routines associated with the operations of the data processing unit (and/or display device) using for example one or more state machines and buffers.

As can be seen in the embodiment of FIG. 12, the analyte sensor 101 (FIG. 11) includes four contacts, three of which are electrodes: a working electrode (W) 210, a reference electrode (R) 212, and a counter electrode (C) 213, each operatively coupled to the analog interface 201 of the data processing unit 102. This embodiment also shows an optional guard contact (G) 211. Fewer or greater electrodes may be employed, without departing from the scope of the present disclosure. For example, the counter and reference electrode functions may be served by a single counter/reference electrode. In some embodiments, there may be more than one working electrode and/or reference electrode and/or counter electrode.

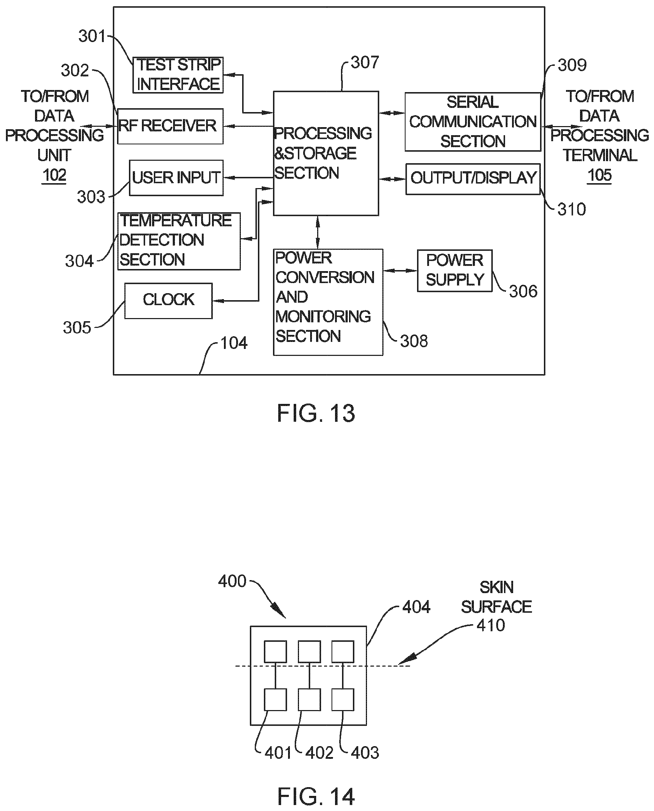

FIG. 13 is a block diagram of an embodiment of a receiver/monitor unit such as the primary display device 104 of the analyte monitoring system shown in FIG. 11. The primary display device 104 includes one or more of: a test strip interface 301, an RF receiver 302, a user input 303, an optional temperature detection section 304, and a clock 305, each of which is operatively coupled to a processing and storage section 307 (that can include processing circuitry and non-transitory memory storing software instructions for execution by the processing circuitry). The primary display device 104 also includes a power supply 306 operatively coupled to a power conversion and monitoring section 308. Further, the power conversion and monitoring section 308 is also coupled to the processing and storage section 307. Moreover, also shown are a receiver serial communication section 309, and an output 310, each operatively coupled to the processing and storage section 307. The primary display device 104 may include user input and/or interface components (e.g., the computing device having the display screens described above) or may be free of user input and/or interface components.

In some embodiments, the test strip interface 301 includes an analyte testing portion (e.g., a glucose level testing portion) to receive a blood (or other body fluid sample) analyte test or information related thereto. For example, the test strip interface 301 may include a test strip port to receive a test strip *(e.g.,* a glucose test strip). The device may determine the analyte level of the test strip, and optionally display (or otherwise notice) the analyte level on the output 310 of the primary display device 104. Any suitable test strip may be employed, such as test strips that only require a very small amount (e.g., 3 microliters or less; *e.g.,* 1 microliter or less; *e.g.,* 0.5 microliters or less; *e.g., 0.1* microliters or less) of applied sample to the strip in order to obtain accurate glucose information. Glucose information obtained by an in vitro glucose testing device may be used for a variety of purposes, computations, and the like. For example, the information may be used to calibrate sensor 101 (FIG. 11), confirm results of sensor 101 to increase the confidence thereof (e.g., in instances in which information obtained by sensor 101 is employed in therapy related decisions), and the like.

In further embodiments, the data processing unit 102 and/or the primary display device 104 and/or the secondary display device 106, and/or the data processing terminal/infusion device 105 may be configured to receive the analyte value wirelessly over a communication link from, for example, a blood glucose meter. In further embodiments, a user manipulating or using the analyte monitoring system 100 may manually input the analyte value using, for example, a user interface (for example, a keyboard, keypad, touchscreen, voice commands, and the like) incorporated in one or more of the data processing unit 102, the primary display device 104, secondary display device 106, and/or the data processing terminal/infusion device 105.

FIG. 14 schematically shows an embodiment of an analyte sensor 400 in accordance with one or more embodiments of the present disclosure. As depicted in FIG. 14, the sensor may include electrodes 401, 402 and 403 on a base 404. Electrodes (and/or other features) may be applied or otherwise processed using any suitable technology, such as chemical vapor deposition (CVD), physical vapor deposition, sputtering, reactive sputtering, printing, coating, ablating (e.g., laser ablation), painting, dip coating, etching, and the like. Materials include, but are not limited to, any one or more of aluminum, carbon (including graphite), cobalt, copper, gallium, gold, indium, iridium, iron, lead, magnesium, mercury (as an amalgam), nickel, niobium, osmium, palladium, platinum, rhenium, rhodium, selenium, silicon (e.g., doped polycrystalline silicon), silver, tantalum, tin, titanium, tungsten, uranium, vanadium, zinc, zirconium, mixtures thereof, and alloys, oxides, or metallic compounds of these elements.

The analyte sensor 400 may be wholly implantable in a user or may be configured so that only a portion is positioned within (internal) a user and another portion outside (external) a user. For example, the sensor 400 may include a first portion positionable above a surface of the skin 410, and a second portion positioned below the surface of the skin. In such embodiments, the external portion may include contacts (connected to respective electrodes of the second portion by traces) to connect to another device also external to the user such as a sensor control device. While the embodiment of FIG. 14 shows three (3) electrodes side-by-side on the same surface of base 404, other configurations are contemplated, including, but not limited to, fewer or greater electrodes, some or all electrodes on different surfaces of the base or present on another base, some or all electrodes stacked together, electrodes of differing materials and dimensions, and the like.

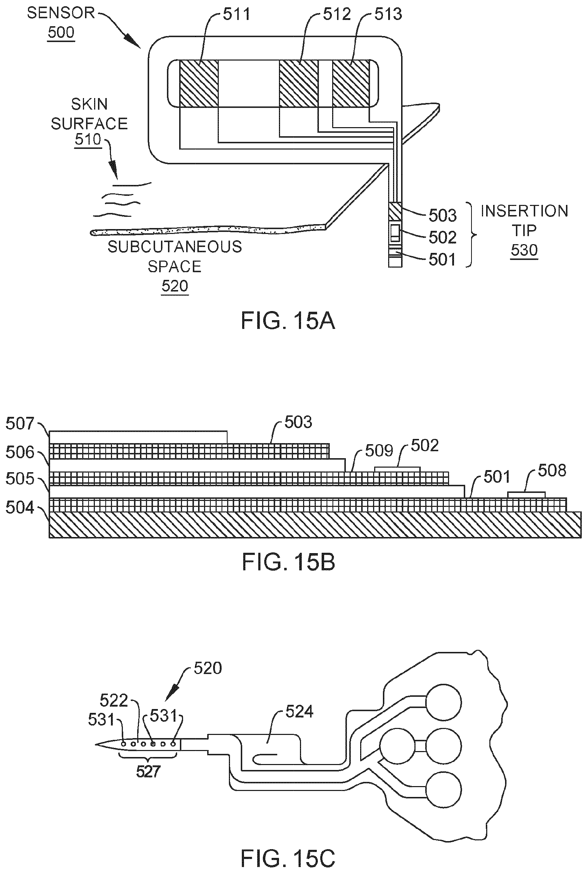

FIG. 15A shows a perspective view of an embodiment of an analyte sensor 500 having a first portion (which in this embodiment may be characterized as a major portion) positionable above a surface of the skin 510, and a second portion (which in this embodiment may be characterized as a minor portion) that includes an insertion tip 530 positionable below the surface of the skin (e.g., penetrating through the skin and into the subcutaneous space 520) and in contact with the user's biofluid, such as interstitial fluid. Contact portions of a working electrode 511, a reference electrode 512, and a counter electrode 513 are positioned on the first portion of the sensor 500 situated above the skin surface 510. A working electrode 501, a reference electrode 502, and a counter electrode 503 are shown at the second portion of the sensor 500 and particularly at the insertion tip 530. Traces may be provided from the electrodes at the tip 530 to the contacts, as shown in FIG. 15A. It is to be understood that greater or fewer electrodes may be provided on a sensor, without departing from the scope of the present disclosure. For example, a sensor may include more than one working electrode and/or the counter and reference electrodes may be a single counter/reference electrode, and the like.

FIG. 15B shows a cross sectional view of a portion of the sensor 500 of FIG. 15A. The electrodes 501, 509/502 and 503, of the sensor 500 as well as the substrate and the dielectric layers are provided in a layered configuration or construction. For example, as shown in FIG. 15B, in an embodiment, the sensor 500 (such as the analyte sensor 101 of FIG. 11), includes a substrate layer 504 and a first conducting layer 501, such as carbon, gold, etc., disposed on at least a portion of the substrate layer 504, which may provide the working electrode. Also shown disposed on at least a portion of the first conducting layer 501 is a sensing region 508.

A first insulation layer 505, such as a first dielectric layer in some embodiments, is disposed or layered on at least a portion of the first conducting layer 501, and further, a second conducting layer 509 may be disposed or stacked on top of at least a portion of the first insulation layer (or dielectric layer) 505. As shown in FIG. 15B, the second conducting layer 509 in conjunction with a second conducting material 502, such as a layer of silver/silver chloride (Ag/AgCl), may provide the reference electrode (e.g., together 509 and 502 may form the reference electrode).

A second insulation layer 506, such as a second dielectric layer in some embodiments, may be disposed or layered on at least a portion of the second conducting layer 509. Further, a third conducting layer 503 may be disposed on at least a portion of the second insulation layer 506 and may provide the counter electrode 503. Finally, a third insulation layer 507 may be disposed or layered on at least a portion of the third conducting layer 503. In this manner, the sensor 500 may be layered such that at least a portion of each of the conducting layers is separated by a respective insulation layer (for example, a dielectric layer). The embodiments of FIGS. 15A and 15B show the layers having different lengths; however, some or all of the layers may have the same or different lengths and/or widths, without departing from the scope of the present disclosure.

In some embodiments, some or all of the electrodes 501, 502, 503 may be provided on the same side of the substrate 504 in the layered construction as described above, or alternatively, may be provided in a co-planar manner such that two or more electrodes may be positioned on the same plane (e.g., side-by side, parallel, or angled relative to each other) on the substrate 504. For example, co-planar electrodes may include a suitable spacing therebetween and/or include a dielectric material or insulation material disposed between the conducting layers/electrodes. Furthermore, in some embodiments, one or more of the electrodes 501, 502, 503 may be disposed on opposing sides of the substrate 504. In such embodiments, contact pads may be on the same or different sides of the substrate. For example, an electrode may be on a first side and its respective contact may be on a second side, for example, a trace connecting the electrode and the contact may traverse through the substrate.

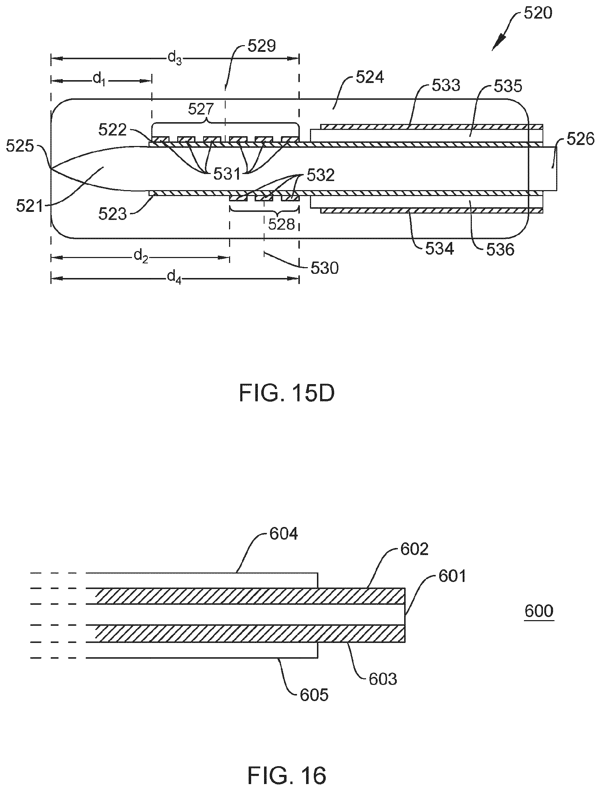

With reference now to FIGS. 15C and 15D, shown is another embodiment of an analyte monitoring sensor in accordance with one or more embodiments of the present disclosure, and representing a variation of the sensor 500 of FIG. 15A. As shown in FIG. 15C and 15D, a transcutaneous sensor 520 according to one or more embodiments of the present disclosure includes a substrate 521, a first working electrode 522 on the substrate 521, a second working electrode 523 on the substrate 521, and a sensor membrane 524 covering the substrate 421 and the first and second working electrodes 522, 523. Although in the illustrated embodiment the first and second working electrodes 522, 523 are positioned on opposite sides of the substrate 511, in one or more embodiments the first and second working electrodes 522, 523 may be positioned in any other suitable locations on the substrate 521. For example, in one or more embodiments, the first and second working electrodes 522, 523 may be on the same side of the substrate 521. The substrate 521 includes a distal end 525 configured to be inserted into the skin of a user and a proximal end 526 opposite the distal end 525 configured to be connected to various electrical connections for transmitting the output signals of the transcutaneous sensor 520. The distal end 525 can have a pointed or rounded tip, or other shaped tips that facilitate insertion of the sensor 520 into the user's skin.

With continued reference to the embodiment illustrated in FIGS. 1B, the first working electrode 522 may include a first active sensing area 527 and the second working electrode 523 may include a second active sensing area 528. Although not shown, the first active sensing area 527 of the first working electrode 522 is configured to transduce an analyte signal into a first output signal (e.g., a current output signal) and the second active sensing area 528 of the second working electrode 523 is configured to transduce an analyte signal into a second output signal (e.g., a current output signal). The output signals of the first and second active sensing areas 527, 528 correspond to a physiological condition of the user, such as, for instance, the blood glucose level of the user. Additionally, in the illustrated embodiment, the first active sensing area 527 of the first working electrode 522 has a first area and the second active sensing area 528 of the second working electrode 523 has a second area, which may be the same or different.

The first active sensing area 527 of the first working electrode 522 is longitudinally offset along the substrate 521 from the second active sensing area 528 of the second working electrode 523. In the illustrated embodiment, a distalmost end of the first active sensing area 527 is spaced apart from the distal end 525 of the substrate 521 by a first distance d1 and a distalmost end of the second active sensing area 528 is spaced apart from the distal end 525 of the substrate 521 by a second distance d2 greater than the first distance d1 *(i.e.,* the distalmost end of the second active sensing area 528 is spaced apart from the distal end 525 of the substrate 521 by a greater distance than the distalmost end of the first active sensing area 527). Additionally, in the illustrated embodiment, a proximalmost end of the first active sensing area 527 is spaced apart from the distal end 525 of the substrate by a third distance d3 and a proximalmost end of the second active sensing area 528 is spaced apart from the distal end 525 of the substrate 521 by a fourth distance d4 that is equal or substantially equal to the third distance d3 *(i.e.,* the proximalmost ends of the first and second active sensing areas 527, 528 are spaced apart from the distal end 525 of the substrate 521 by the same or substantially the same distance). Accordingly, in the illustrated embodiment, a longitudinally central portion 529 of the first active sensing area 527 is offset from a longitudinally central portion 530 of the second active sensing area 528. In one or more embodiments, the proximalmost end of the first active sensing area 527 may not be aligned with the proximalmost end of the second active sensing area 528.

Additionally, in the illustrated embodiment, the first area of the first active sensing area 527 is greater than the second area of the second active sensing area 528. In the illustrated embodiment, the first and second active sensing areas 527, 528 each include a series of discrete sensing spots 531, 532 (*e.g*., dots), respectively. In the illustrated embodiment, the size of each of the discrete sensing spots 531 in the first active sensing area 527 is equal or substantially equal to the size of each of the discrete sensing spots 532 in the second active sensing area 528. In a preferred embodiment, the number of discrete sensing spots 531 in the first active sensing area 527 is greater than the number of discrete spots 532 in the second active sensing area 528; however, in other embodiments the discrete sensing spots 531, 532 may be equal in number or there may be less discrete sensing spots 531 than discrete sensing spots 532, without departing from the scope of the present disclosure. Although in the illustrated embodiment there are six (6) uniformly sized discrete sensing spots 531 in the first active sensing area 527 and three (3) uniformly sized discrete sensing spots 532 in the second active sensing area 528, in one or more embodiments, the first and second active sensing areas 527, 528 may include any other suitable number of discrete sensing spots, without departing from the scope of the present disclosure. Additionally, in one or more embodiments, the first active sensing area 527 and/or the second active sensing area 528 may include a continuous strip (e.g., an elongated ellipse) rather than a series of discrete sensing spots. Furthermore, in one or more embodiments, the first area of the first active sensing area 527 may be equal or substantially equal to the second area of the second active sensing area 528.

Additionally, in one or more embodiments, transcutaneous sensor 520 may include a reference electrode, a counter electrode, or counter-reference electrodes. In the illustrated embodiment, the transcutaneous sensor 520 includes a counter electrode 533 and a reference electrode 534. In the illustrated embodiment, the reference electrode 534 and the counter electrode 533 are on opposite sides of the substrate 521, but may be on the same side of the substrate 521, without departing from the scope of the present disclosure. Additionally, in the illustrated embodiment, the counter electrode 533 is separated from the first working electrode 522 by a first dielectric insulator layer 535 and the reference electrode 534 is separated from the second working electrode 523 by a second dielectric insulator layer 536.

Embodiments of a double-sided, stacked sensor configuration which may be utilized in connection with the present disclosure are described herein with reference to FIGS. 16-18. FIG. 16 shows a cross-sectional view of a distal portion of a double-sided analyte sensor 600. Analyte sensor 600 includes an at least generally planar insulative base substrate 601, *e.g.,* an at least generally planar dielectric base substrate, having a first conductive layer 602 which substantially covers the entirety of a first surface area, e.g., the top surface area, of insulative substrate 601, e.g., the conductive layer substantially extends the entire length of the substrate to the distal edge and across the entire width of the substrate from side edge to side edge. A second conductive layer 603 substantially covers the entirety of a second surface, e.g., the bottom side, of insulative base substrate 601. However, one or both of the conductive layers may terminate proximally of the distal edge and/or may have a width that is less than that of insulative substrate 601 where the width ends a selected distance from the side edges of the substrate, which distance may be equidistant or vary from each of the side edges.

One of the first or second conductive layers, e.g., first conductive layer 602, may be configured to include the sensor's working electrode. The opposing conductive layer, here, second conductive layer 603, may be configured to include a reference and/or counter electrode. Where conductive layer 603 serves as either a reference or counter electrode, but not both, a third electrode may optionally be provided either on a surface area of the proximal portion of the sensor (not shown), on a separate substrate, or as an additional conductive layer positioned either above or below conductive layer 602 or 603 and separated from those layers by an insulative layer or layers. For example, in some embodiments, where analyte sensor 600 is configured to be partially implanted, conductive layer 603 may be configured to include a reference electrode, and a third electrode (not shown) and present only on a non-implanted proximal portion of the sensor may be configured to include the sensor's counter electrode.

A first insulative layer 604 covers at least a portion of conductive layer 602 and a second insulative layer 605 covers at least a portion of conductive layer 603. In one embodiment, at least one of first insulative layer 604 and second insulative layer 605 does not extend to the distal end of analyte sensor 600 leaving an exposed region of the conductive layer or layers.

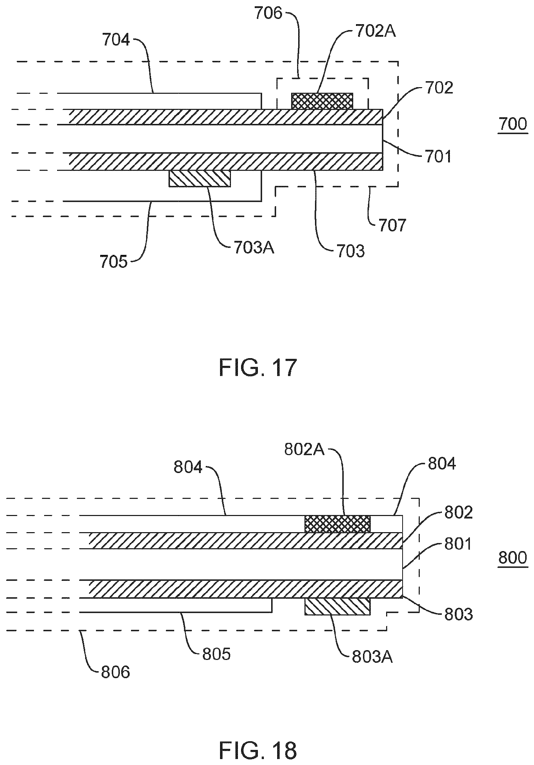

FIG. 17 shows a cross-sectional view of a distal portion of a double-sided analyte sensor 700 including an at least generally planar insulative base substrate 701, *e.g.,* an at least generally planar dielectric base substrate, having a first conductive layer 702 which substantially covers the entirety of a first surface area, *e.g.,* the top surface area, of insulative substrate 701, *e.g.,* the conductive layer substantially extends the entire length of the substrate to the distal edge and across the entire width of the substrate from side edge to side edge. A second conductive layer 703 substantially covers the entirety of a second surface, e.g., the bottom side, of insulative base substrate 701. However, one or both of the conductive layers may terminate proximally of the distal edge and/or may have a width that is less than that of insulative substrate 701 where the width ends a selected distance from the side edges of the substrate, which distance may be equidistant or vary from each of the side edges.

In the embodiment of FIG. 17, conductive layer 702 is configured to include a working electrode which includes a sensing region 702A disposed on at least a portion of the first conductive layer 702 as shown and as discussed in greater detail below. While a single sensing region 702A is shown, it should be noted that in other embodiments a plurality of spatially separated sensing elements may be utilized, without departing from the scope of the present disclosure.

In the embodiment of FIG. 17, conductive layer 703 is configured to include a reference electrode which includes a secondary layer of conductive material 703A, e.g., Ag/AgCl, disposed over a distal portion of conductive layer 703.

A first insulative layer 704 covers a portion of conductive layer 702 and a second insulative layer 705 covers a portion of conductive layer 703. First insulative layer 704 does not extend to the distal end of analyte sensor 700, leaving an exposed region of the conductive layer where the sensing region 702A is positioned. The insulative layer 705 on the bottom/reference electrode side of the sensor may extend any suitable length of the sensor's distal section, e.g., it may extend the entire length of both of the primary and secondary conductive layers or portions thereof. For example, as illustrated in FIG. 17, bottom insulative layer 705 extends over the entire bottom surface area of secondary conductive material 703A but terminates proximally of the distal end of the length of the conductive layer 703. It is noted that at least the ends of the secondary conductive material 703A that extend along the side edges of the substrate 701 are not covered by insulative layer 705 and, as such, are exposed to the environment when in operative use.

In an alternative embodiment, as shown in FIG. 18, analyte sensor 800 has an insulative layer 804 on the working electrode side of an insulative base substrate 801, which may be provided prior to sensing region 802A whereby the insulative layer 804 has at least two portions spaced apart from each other on conductive layer 802. The sensing region 802A is then provided in the spacing between the two portions. More than two spaced apart portions may be provided, e.g., where a plurality of sensing components or layers is desired. Bottom insulative layer 805 has a length which terminates proximally of secondary conductive layer 803A on bottom primary conductive layer 803. Additional conducting and dielectric layers may be provided on either or both sides of the sensors, as described above.

While FIGS. 16-18 depict or are discussed herein as capable of providing the working and reference electrodes in a particular layered configuration, it should be noted that the relative positioning of these layers may be modified. For example, a counter electrode layer may be provided on one side of an insulative base substrate while working and reference electrode layers are provided in a stacked configuration on the opposite side of the insulative base substrate. In addition, a different number of electrodes may be provided than depicted in FIGS. 16-18 by adjusting the number of conductive and insulative layers. For example, a three (3) or four (4) electrode sensor may be provided.

One or more membranes, which may function as one or more of an analyte flux modulating layer and/or an interferent-eliminating layer and/or biocompatible layer, discussed in greater detail below, may be included with, on, or about the sensor (e.g., as one or more of the outermost layer(s)). The membrane of the present disclosure may take many forms. For example, the membrane may include just one component, or multiple components. The membrane may have a globular shape, such as if encompassing a terminal region of the sensor (e.g., the lateral sides and terminal tip). The membrane may have a generally planar structure, and can be characterized as a layer. Planar membranes may be smooth or may have minor surface (topological) variations. The membrane may also be configured as other non-planar structures. For example, the membrane may have a cylindrical shape or a partially cylindrical shape, a hemispherical shape or other partially spherical shape, an irregular shape, or other rounded or curved shape.

In some embodiments, as illustrated in FIG. 17, a first membrane layer 706 may be provided solely over the sensing region 702A on the working electrode 702 to modulate the rate of diffusion or flux of the analyte to the sensing region. For embodiments in which a membrane layer is provided over a single component/material, it may be suitable to do so with the same striping configuration and method as used for the other materials/components. Here, the membrane material 706 preferably has a width greater than that of sensing component 702A. As it acts to limit the flux of the analyte to the sensor's active area, and thus contributes to the sensitivity of the sensor, controlling the thickness of membrane 706 is important. Providing membrane 706 in the form of a stripe/band facilitates control of its thickness. A second membrane layer 707, which coats the remaining surface area of the sensor tail, may also be provided to serve as a biocompatible conformal coating and provide smooth edges over the entirety of the sensor.

In other sensor embodiments, as illustrated in FIG. 18, a single, homogenous membrane 806 may be coated over the entire sensor surface area, or at least over both sides of the distal tail portion. It is noted that to coat the distal and side edges of the sensor, the membrane material may have to be applied subsequent to singulation of the sensor precursors. In some embodiments, the analyte sensor is dip-coated following singulation to apply one or more membranes. Alternatively, the analyte sensor may be slot-die coated, wherein each side of the analyte sensor is coated separately.

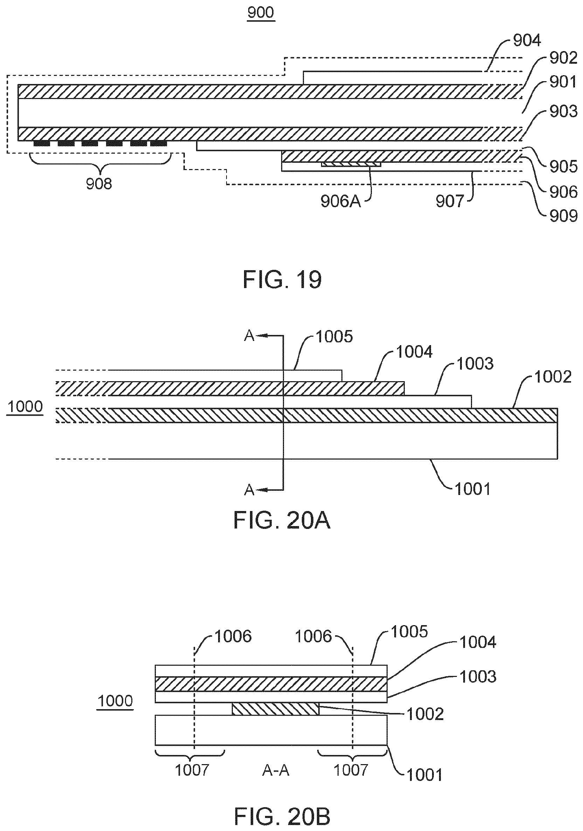

FIG. 19 shows a cross-sectional view of a distal portion of an example double-sided analyte sensor 900 according to one embodiment of the present disclosure, wherein the double-sided analyte sensor includes an at least generally planar insulative base substrate 901, *e.g.,* an at least generally planar dielectric base substrate, having a first conductive layer 902. A second conductive layer 903 is positioned on a first side, e.g., the bottom side, of insulative base substrate 901. While depicted as extending to the distal edge of the sensor, one or both of the conductive layers may terminate proximally of the distal edge and/or may have a width which is less than that of insulative substrate 901 where the width ends a selected distance from the side edges of the substrate, which distance may be equidistant or vary from each of the side edges. For example, the first and second conductive layers may be provided which define electrodes, including, e.g., electrode traces, which have widths that are less than that of the insulative base substrate.

In the embodiment of FIG. 19, conductive layer 903 is configured to include a working electrode which includes a sensing region 908 disposed on at least a portion of the conductive layer 903, which sensing region is discussed in greater detail below. It should be noted that a plurality of spatially separated sensing components or layers may be utilized in forming the working electrode, e.g., one or more discrete sensing spots or "dots" or areas may be provided on the conductive layer 903, as shown herein, or a single sensing component may be used (not shown).

In the embodiment of FIG. 19, conductive layer 906 is configured to include a reference electrode which includes a secondary layer of conductive material 906A, e.g., Ag/AgCl, disposed on a distal portion of conductive layer 906. Like conductive layers 902 and 903, conductive layer 906 may terminate proximally of the distal edge and/or may have a width that is less than that of insulative substrate 901 where the width ends a selected distance from the side edges of the substrate, which distance may be equidistant or vary from each of the side edges, as discussed in greater detail below in reference to FIGS. 20A-20C

In the embodiment shown in FIG. 19, conductive layer 902 is configured to include a counter electrode. A first insulative layer 904 covers a portion of conductive layer 902 and a second insulative layer 905 covers a portion of conductive layer 903. First insulative layer 904 does not extend to the distal end of analyte sensor 900 leaving an exposed region of the conductive layer 902 that acts as the counter electrode. An insulative layer 905 covers a portion of conductive layer 903 leaving an exposed region of the conductive layer 903 where the sensing region 908 is positioned. As discussed above, multiple spatially separated sensing components or layers may be provided (as shown) in some embodiments, while in other embodiments a single sensing region may be provided, without departing from the scope of the present disclosure. The insulative layer 907 on a first side, e.g., the bottom side of the sensor (in the view provided by FIG. 19), may extend any suitable length of the sensor's distal section, *e.g.,* it may extend the entire length of both of conductive layers 906 and 906A or portions thereof. For example, as illustrated in FIG. 19, bottom insulative layer 907 extends over the entire bottom surface area of secondary conductive material 906A and terminates distally of the distal end of the length of the conductive layer 906. It is noted that at least the ends of the secondary conductive material 906A that extend along the side edges of the substrate 901 are not covered by insulative layer 907 and, as such, are exposed to the environment when in operative use.

As illustrated in FIG. 19, a homogenous membrane 909 may be coated over the entire sensor surface area, or at least over both sides of the distal tail portion. It is noted that to coat the distal and side edges of the sensor, the membrane material may have to be applied subsequent to singulation of the sensor precursors. In some embodiments, the analyte sensor is dip-coated following singulation to apply one or more membranes (or to apply one membrane in various stages). Alternatively, the analyte sensor may be slot-die coated wherein each side of the analyte sensor is coated separately. Membrane 909 is shown in FIG. 19 as having a squared shape matching the underlying surface variations, but can have a more globular or amorphous shape, as well.

When manufacturing layered sensors, it may be desirable to utilize relatively thin insulative layers to reduce total sensor width. For example, with reference to FIG. 19, insulative layers 904, 905 and 907 may be relatively thin relative to insulative substrate layer 901. For example, insulative layers 904, 905 and 907 may have a thickness in the range of 20-25 micrometers (µm) while substrate layer 901 may have a thickness in the range of 0.1 to 0.15 millimeters (mm). However, during singulation of the sensors where such singulation is accomplished by cutting through two or more conductive layers which are separated by such thin insulative layers, shorting between the two conductive layers may occur.

One method of addressing this potential issue is to provide one of the conductive layers, e.g., electrodes layers, at least in part as a relatively narrow electrode, including, e.g., a relatively narrow conductive trace, such that during the singulation process the sensor is cut on either side of the narrow electrode such that one electrode is cut without cutting through the narrow electrode.

For example, with reference to FIGS. 20A-20C, a sensor 1000 is depicted which includes insulative layers 1003 and 1005. Insulative layers 1003 and 1005 may be thin relative to generally planar insulative base substrate layer 1001, or vice versa. For example, insulative layers 1003 and 1005 may have a thickness in the range of 15-30 µm while substrate layer 1001 has a thickness in the range of 0.1 to 0.15 mm. Such sensors may be manufactured in sheets wherein a single sheet includes a plurality of sensors. However, such a process generally requires singulation of the sensors prior to use. Where such singulation requires cutting through two or more conductive layers which are separated by insulative layers, shorting between the two conductive layers may occur, particularly if the insulative layers are thin. In order to avoid such shorting, fewer than all of the conductive layers may be cut through during the singulation process. For example, at least one of the conductive layers may be provided at least in part as an electrode, e.g., including a conductive trace, having a narrow width relative to one or more other conductive layers such that during the singulation process a first conductive layer separated from a second conductive layer only by a thin insulative layer, e.g., an insulative layer having a thickness in the range of 15-30 µm, is cut while a second conductive layer is not.

With continued reference to FIGS. 20A and 20C, sensor 1000 includes an at least generally planar insulative base substrate 1001. Positioned on the at least generally planar insulative base substrate 1001 is a first conductive layer 1002. A first relatively thin insulative layer 1003, *e.g.,* an insulative layer having a thickness in the range of 15-30 µm, is positioned on the first conductive layer 1002 and second conductive layer 1004 is positioned on the relatively thin insulative layer 1003. Finally, a second relatively thin insulative layer 1005, *e.g.,* an insulative layer having a thickness in the range of 15-30 µm, is positioned on the second conductive layer 1004.

As shown in FIG. 20B, first conductive layer 1002 may be an electrode having a narrow width relative to conductive layer 1004 as shown in the FIG. 20B cross-section taken at lines A-A. Alternatively, second conductive layer 1004 may be a conductive electrode having a narrow width relative to conductive layer 1002 shown in the FIG. 20C cross-section taken at lines A-A. Singulation cut lines 1006 are shown in FIGS. 20B and 20C. The sensor may be singulated, for example, by cutting to either side of the relatively narrow conductive electrode, *e.g.,* in regions 1007, as shown in FIGS. 20B and 20C. With reference to FIG. 20B, singulation by cutting along singulation cut lines 1006 results in cutting through conductive layer 1004 but not conductive layer 1002. With reference to FIG. 20C, singulation by cutting along singulation cut lines 1006 results in cutting through conductive layer 1002 but not conductive layer 1004.

An embodiment of a sensing region may be described as the area shown schematically in FIG. 115B as 508 and FIG. 9 as 908. As noted above the sensing region may be provided as a single sensing component as shown in FIG. 15B as 508, FIG. 17 as 702A and FIG. 18 as 802A, or provided as a plurality of sensing components as shown in FIG. 19 as 908. A plurality of sensing components or sensing "spots" is described in U.S. Patent Application Publication No. 2012/0150005, incorporated by reference herein in its entirety.

As used herein, the term "sensing region," and grammatical variants thereof, is a broad term and may be described as the active chemical area of an analyte monitoring sensor or biosensor. The sensing region may take many forms. The sensing region may include just one component, or multiple components (e.g., such as sensing region 908 of FIG. 19). In the embodiment of FIG. 15B, for example, the sensing region is a generally planar structure, and can be characterized as a layer. Planar sensing regions can be smooth or can have minor surface (topological) variations. The sensing region may also be a non-planar structure. For example, the sensing region can have a cylindrical shape or a partially cylindrical shape, a hemispherical shape or other partially spherical shape, an irregular shape, or other rounded or curved shape.

The sensing region formulation, which can include a glucose-transducing agent, may include, for example, among other constituents, a redox mediator, such as, for example, a hydrogen peroxide or a transition metal complex, such as a ruthenium-containing complex or an osmium-containing complex, and an analyte-responsive enzyme, such as, for example, a glucose-responsive enzyme (e.g., glucose oxidase, glucose dehydrogenase, etc.) or lactate-responsive enzyme (e.g., lactate oxidase). In some embodiments, the sensing region includes glucose oxidase. The sensing region may also include other optional components, such as, for example, a polymer and a bi-functional, short-chain, epoxide cross-linker, such as polyethylene glycol (PEG).

In some embodiments, the sensing region formulation includes protein switch components that permit detection of any desired analyte. Use of a protein switch allows a selected redox mediator, such as, for example, a hydrogen peroxide or a transition metal complex, such as a ruthenium-containing complex or an osmium-containing complex, coupled to a selected enzyme, such as, for example, a glucose-responsive enzyme (e.g., glucose oxidase, glucose dehydrogenase, and the like) or lactate-responsive enzyme (e.g., lactate oxidase) to provide a qualitative or quantitative detection platform for any desired analyte. The selected enzyme is coupled (e.g., covalently linked) to a selective analyte-binding ligand (e.g., peptide, antibody, antibody fragment, other immunoglobulin, apatamer, and the like) such that binding of the analyte-binding ligand by an analyte present in an analyzed sample alters (e.g., inhibits or enhances) the activity of the selected enzyme. The presence of the analyte in an analyzed sample thereby increases or decreases, as desired, with a detectable product of the enzyme activity (e.g., change in redox state of the reaction solution). While certain examples of a selected enzyme component of a protein switch are described herein, it should be understood that any enzyme, or enzymatically functional portion thereof, that catalyzes production of a product that can be detected (e.g., electrochemically detected) may be employed. Any of a wide variety of analytes may be detected using such a system, including, but not limited to, proteins and peptide, lipids, carbohydrates, metabolites, hormones, synthetic molecules (e.g., drugs) or metabolized products thereof, antibodies, pathogen components, nucleic acids, toxins, minerals, and the like. The analyte binding part of the protein switch can be derived from a protein that binds to the analyte. Such proteins that bind the analyte can include, for example, antibodies, receptors (including full length, fragments, and single chain receptors), and artificial binding proteins made using scaffolds or display technologies. Alternatively, the analyte binding part can be derived from a ligand when the analyte to be detected is a receptor or derived from a receptor.

A protein switch can be derived from a protein that has a binding affinity for the analyte which can allow the protein switch to detect analyte at physiological levels. The protein switch can be made from an analyte binding protein that has desired kinetics for binding of physiological levels of the analyte. Specific example protein switch components and methods of designing, making, enhancing, and optimizing protein switch components (e.g., using libraries of fusion proteins and high throughput screening technologies) for a wide variety of analytes are described in U.S. Provisional Patent Application Serial Number 62/468,878 (filed March 8, 2017), and U.S. Provisional Patent Application Serial Number 62/544,364 (filed August 11, 2017), both of which are incorporated by reference herein in their entireties and for all purposes.

In some embodiments, two or more different protein switch systems are employed in a single sensor that are responsive to two or more different analytes. In some such embodiments, the different analytes generate the same reporter signal in the same region such that the presence of any analyte produces the detectable result. In other embodiments, the different analytes generate different or distinguishable signals so that each analyte may be separately detected and analyzed (e.g., generating different signals or generating the same signal in different regions (e.g., different layers of a multi-layer sensor)).

In certain instances, the analyte-responsive enzyme is distributed throughout the sensing region. For example, the analyte-responsive enzyme may be distributed uniformly throughout the sensing region, such that the concentration of the analyte-responsive enzyme is substantially the same throughout the sensing region. In some cases, the sensing region may have a homogeneous distribution of the analyte-responsive enzyme. In some embodiments, the redox mediator is distributed throughout the sensing region. For example, the redox mediator may be distributed uniformly throughout the sensing region, such that the concentration of the redox mediator is substantially the same throughout the sensing region. In some cases, the sensing region may have a homogeneous distribution of the redox mediator. In some embodiments, both the analyte-responsive enzyme and the redox mediator are distributed uniformly throughout the sensing region, as described above.

As noted above, analyte sensors may include an analyte-responsive enzyme to provide a sensing component or sensing region. Some analytes, such as oxygen, can be directly electrooxidized or electroreduced on a sensor, and more specifically at least on a working electrode of a sensor. Other analytes, such as glucose and lactate, require the presence of at least one electron transfer agent and/or at least one catalyst to facilitate the electrooxidation or electroreduction of the analyte. Catalysts may also be used for those analytes, such as oxygen, that can be directly electrooxidized or electroreduced on the working electrode. For these analytes, each working electrode includes a sensing region (see for example sensing region 508 of FIG. 15B) proximate to or on a surface of a working electrode. In many embodiments, a sensing region is formed near or on only a small portion of at least a working electrode.

The sensing region can include one or more components constructed to facilitate the electrochemical oxidation or reduction of the analyte. The sensing region may include, for example, a catalyst to catalyze a reaction of the analyte and produce a response at the working electrode, an electron transfer agent to transfer electrons between the analyte and the working electrode (or other component), or both.

A variety of different sensing region configurations may be used in the embodiments of the present disclosure. The sensing region is often located in contact with or in proximity to an electrode, such as the working electrode. In some embodiments, the sensing region is deposited on the conductive material of the working electrode. The sensing region may extend beyond the conductive material of the working electrode. In some cases, the sensing region may also extend over other electrodes, e.g., over the counter electrode and/or reference electrode (or if a counter/reference is provided).

A sensing region that is in direct contact with the working electrode may contain an electron transfer agent to transfer electrons directly or indirectly between the analyte and the working electrode, and/or a catalyst to facilitate a reaction of the analyte. For example, a glucose, lactate, or oxygen electrode may be formed having a sensing region which contains a catalyst, including glucose oxidase, glucose dehydrogenase, lactate oxidase, or laccase, respectively, and an electron transfer agent that facilitates the electrooxidation of the glucose, lactate, or oxygen, respectively. As described above, a protein switch may be employed, providing an indirect mechanism for detecting an analyte of interest by translating the binding of an analyte to a binding partner to a change in activity of an enzyme.

In other embodiments, the sensing region is not deposited directly on the working electrode. Instead, the sensing region 508 (FIG. 15), for example, may be spaced apart from the working electrode, and separated from the working electrode, e.g., by a separation layer. A separation layer may include one or more membranes or films or a physical distance. In addition to separating the working electrode from the sensing region, the separation layer may also act as a mass transport limiting layer and/or an interferent eliminating layer and/or a biocompatible layer.

In some embodiments which include more than one working electrode, one or more of the working electrodes may not have a corresponding sensing region, or may have a sensing region which does not contain one or more components (e.g., an electron transfer agent and/or catalyst) needed to electrolyze the analyte. Thus, the signal at this working electrode may correspond to background signal which may be removed from the analyte signal obtained from one or more other working electrodes that are associated with fully-functional sensing regions by, for example, subtracting the signal.

In some embodiments, the sensing region includes one or more electron transfer agents. Electron transfer agents that may be employed are electroreducible and electrooxidizable ions or molecules having redox potentials that are a few hundred millivolts above or below the redox potential of the standard calomel electrode (SCE). The electron transfer agent may be organic, organometallic, or inorganic. Examples of organic redox species are quinones and species that in their oxidized state have quinoid structures, such as Nile blue and indophenol. Examples of organometallic redox species are metallocenes including ferrocene. Examples of inorganic redox species are hexacyanoferrate (III), ruthenium hexamine, and the like. Additional examples include those described in U.S. Patent Nos. 6,736,957, 7,501,053 and 7,754,093, the disclosures of each of which are incorporated herein by reference in their entirety.

In some embodiments, electron transfer agents have structures or charges which prevent or substantially reduce the diffusional loss of the electron transfer agent during the period of time that the sample is being analyzed. For example, electron transfer agents include but are not limited to a redox species, e.g., bound to a polymer which can in turn be disposed on or near the working electrode. The bond between the redox species and the polymer may be covalent, coordinative, or ionic. Although any organic, organometallic or inorganic redox species may be bound to a polymer and used as an electron transfer agent, in some embodiments the redox species is a transition metal compound or complex, e.g., osmium, ruthenium, iron, and cobalt compounds or complexes. It will be recognized that many redox species described for use with a polymeric component may also be used, without a polymeric component.

Embodiments of polymeric electron transfer agents may contain a redox species covalently bound in a polymeric composition. An example of this type of mediator is poly(vinylferrocene). Another type of electron transfer agent contains an ionically-bound redox species. This type of mediator may include a charged polymer coupled to an oppositely charged redox species. Examples of this type of mediator include a negatively charged polymer coupled to a positively charged redox species such as an osmium or ruthenium polypyridyl cation. Another example of an ionically-bound mediator is a positively charged polymer including quaternized poly(4-vinyl pyridine) or poly(1-vinyl imidazole) coupled to a negatively charged redox species such as ferricyanide or ferrocyanide. In other embodiments, electron transfer agents include a redox species coordinatively bound to a polymer. For example, the mediator may be formed by coordination of an osmium or cobalt 2,2'-bipyridyl complex to poly(1-vinyl imidazole) or poly(4-vinyl pyridine).

Suitable electron transfer agents are osmium transition metal complexes with one or more ligands, each ligand having a nitrogen-containing heterocycle such as 2,2'-bipyridine, 1,10-phenanthroline, 1-methyl, 2-pyridyl biimidazole, or derivatives thereof. The electron transfer agents may also have one or more ligands covalently bound in a polymer, each ligand having at least one nitrogen-containing heterocycle, such as pyridine, imidazole, or derivatives thereof. One example of an electron transfer agent includes (a) a polymer or copolymer having pyridine or imidazole functional groups and (b) osmium cations complexed with two ligands, each ligand containing 2,2'-bipyridine, 1,10-phenanthroline, or derivatives thereof, the two ligands not necessarily being the same. Some derivatives of 2,2'-bipyridine for complexation with the osmium cation include but are not limited to 4,4'-dimethyl-2,2'-bipyridine and mono-, di-, and polyalkoxy-2,2'-bipyridines, including 4,4'-dimethoxy-2,2'-bipyridine. Derivatives of 1,10-phenanthroline for complexation with the osmium cation include but are not limited to 4,7-dimethyl-1,10-phenanthroline and mono, di-, and polyalkoxy-1,10-phenanthrolines, such as 4,7-dimethoxy-1,10-phenanthroline. Polymers for complexation with the osmium cation include but are not limited to polymers and copolymers of poly(1-vinyl imidazole) (referred to as "PVI") and poly(4-vinyl pyridine) (referred to as "PVP"). Suitable copolymer substituents of poly(1-vinyl imidazole) include acrylonitrile, acrylamide, and substituted or quaternized N-vinyl imidazole, e.g., electron transfer agents with osmium complexed to a polymer or copolymer of poly(1-vinyl imidazole).

Embodiments may employ electron transfer agents having a redox potential ranging from about -200 mV to about +200 mV versus the standard calomel electrode (SCE). The sensing region may also include a catalyst which is capable of catalyzing a reaction of the analyte. The catalyst may also, in some embodiments, act as an electron transfer agent. One example of a suitable catalyst is an enzyme which catalyzes a reaction of the analyte. For example, a catalyst, including a glucose oxidase, glucose dehydrogenase *(e.g.,* pyrroloquinoline quinone (PQQ), dependent glucose dehydrogenase, flavine adenine dinucleotide (FAD) dependent glucose dehydrogenase, or nicotinamide adenine dinucleotide (NAD) dependent glucose dehydrogenase), may be used when the analyte of interest is glucose. A lactate oxidase or lactate dehydrogenase may be used when the analyte of interest is lactate. Laccase may be used when the analyte of interest is oxygen or when oxygen is generated or consumed in response to a reaction of the analyte.

In some embodiments, a catalyst may be attached to a polymer, cross linking the catalyst with another electron transfer agent, which, as described above, may be polymeric. A second catalyst may also be used in some embodiments. This second catalyst may be used to catalyze a reaction of a product compound resulting from the catalyzed reaction of the analyte. The second catalyst may operate with an electron transfer agent to electrolyze the product compound to generate a signal at the working electrode. Alternatively, a second catalyst may be provided in an interferent-eliminating layer to catalyze reactions that remove interferents.

In some embodiments, the sensor operates at a low oxidizing potential, e.g., a potential of about +40 mV vs. Ag/AgCl. This sensing region uses, for example, an osmium (Os)-based mediator constructed for low potential operation. Accordingly, in some embodiments the sensing element is a redox active component that includes (1) osmium-based mediator molecules that include (bidente) ligands, and (2) glucose oxidase enzyme molecules. These two constituents are combined together in the sensing region of the sensor.

A mass transport limiting layer (not shown), e.g., an analyte flux modulating layer, may be included with the sensor to act as a diffusion-limiting barrier to reduce the rate of mass transport of the analyte, for example, glucose or lactate, into the region around the working electrodes. The mass transport limiting layers are useful in limiting the flux of an analyte to a working electrode in an electrochemical sensor so that the sensor is linearly responsive over a large range of analyte concentrations and is easily calibrated. Mass transport limiting layers may include polymers and may be biocompatible. A mass transport limiting layer may provide many functions, e.g., biocompatibility and/or interferent-eliminating functions, and the like.

In some embodiments, a mass transport limiting layer is a membrane composed of crosslinked polymers containing heterocyclic nitrogen groups, such as polymers of polyvinylpyridine and polyvinylimidazole. Embodiments also include membranes that are made of a polyurethane, or polyether urethane, or chemically related material, or membranes that are made of silicone, and the like.

A membrane may be formed by crosslinking in situ a polymer, modified with a zwitterionic moiety, a non-pyridine copolymer component, and optionally another moiety that is either hydrophilic or hydrophobic, and/or has other desirable properties, in an alcohol-buffer solution. The modified polymer may be made from a precursor polymer containing heterocyclic nitrogen groups. For example, a precursor polymer may be polyvinylpyridine or polyvinylimidazole. Optionally, hydrophilic or hydrophobic modifiers may be used to "fine-tune" the permeability of the resulting membrane to an analyte of interest. Optional hydrophilic modifiers, such as poly(ethylene glycol), hydroxyl, or polyhydroxyl modifiers, may be used to enhance the biocompatibility of the polymer or the resulting membrane.

A membrane may be formed in situ by applying an alcohol-buffer solution of a crosslinker and a modified polymer over an enzyme-containing sensing region and allowing the solution to cure for about one to two days or other appropriate time period. The crosslinker-polymer solution may be applied to the sensing region by placing a droplet or droplets of the membrane solution on the sensor, by dipping the sensor into the membrane solution, by spraying the membrane solution on the sensor, and the like. Generally, the thickness of the membrane is controlled by the concentration of the membrane solution, by the number of droplets of the membrane solution applied, by the number of times the sensor is dipped in the membrane solution, by the volume of membrane solution sprayed on the sensor, or by any combination of these factors. A membrane applied in this manner may have any combination of the following functions: (1) mass transport limitation, e.g., reduction of the flux of analyte that can reach the sensing region, (2) biocompatibility enhancement, or (3) interferent reduction.

In some instances, the membrane may form one or more bonds with the sensing region. By bonds is meant any type of an interaction between atoms or molecules that allows chemical compounds to form associations with each other, such as, but not limited to, covalent bonds, ionic bonds, dipole-dipole interactions, hydrogen bonds, London dispersion forces, and the like. For example, in situ polymerization of the membrane can form crosslinks between the polymers of the membrane and the polymers in the sensing region. In some embodiments, crosslinking of the membrane to the sensing region facilitates a reduction in the occurrence of delamination of the membrane from the sensing region.

In some embodiments, the sensing system detects hydrogen peroxide to infer glucose levels. For example, a hydrogen peroxide-detecting sensor may be constructed in which a sensing region includes enzyme such as glucose oxides, glucose dehydrogenase, or the like, and is positioned proximate to the working electrode. The sensing region may be covered by one or more layers, e.g., a membrane that is selectively permeable to glucose. Once the glucose passes through the membrane, it is oxidized by the enzyme and reduced glucose oxidase can then be oxidized by reacting with molecular oxygen to produce hydrogen peroxide.

Certain embodiments include a hydrogen peroxide-detecting sensor constructed from a sensing region prepared by combining together, for example: (1) a redox mediator having a transition metal complex including an Os polypyridyl complex with oxidation potentials of about +200 mV vs. SCE, and (2) periodate oxidized horseradish peroxidase (HRP). Such a sensor functions in a reductive mode; the working electrode is controlled at a potential negative to that of the Os complex, resulting in mediated reduction of hydrogen peroxide through the HRP catalyst.

In another example, a potentiometric sensor can be constructed as follows. A glucose-sensing region is constructed by combining together (1) a redox mediator having a transition metal complex including Os polypyridyl complexes with oxidation potentials from about -200 mV to +200 mV vs. SCE, and (2) glucose oxidase. This sensor can then be used in a potentiometric mode, by exposing the sensor to a glucose containing solution, under conditions of zero current flow, and allowing the ratio of reduced/oxidized Os to reach an equilibrium value. The reduced/oxidized Os ratio varies in a reproducible way with the glucose concentration, and will cause the electrode's potential to vary in a similar way.

The substrate may be formed using a variety of non-conducting materials, including, for example, polymeric or plastic materials and ceramic materials. Suitable materials for a particular sensor may be determined, at least in part, based on the desired use of the sensor and properties of the materials.

In some embodiments, the substrate is flexible. For example, if the sensor is configured for implantation into a user, then the sensor may be made flexible (although rigid sensors may also be used for implantable sensors) to reduce pain to the user and damage to the tissue caused by the implantation of and/or the wearing of the sensor. A flexible substrate often increases the user's comfort and allows a wider range of activities. Suitable materials for a flexible substrate include, for example, non-conducting plastic or polymeric materials and other non-conducting, flexible, deformable materials. Examples of useful plastic or polymeric materials include thermoplastics such as polycarbonates, polyesters (e.g., Mylar^{™} and polyethylene terephthalate (PET)), polyvinyl chloride (PVC), polyurethanes, polyethers, polyamides, polyimides, or copolymers of these thermoplastics, such as PETG (glycol-modified polyethylene terephthalate).

In other embodiments, the sensors are made using a relatively rigid substrate to, for example, provide structural support against bending or breaking. Examples of rigid materials that may be used as the substrate include poorly conducting ceramics, such as aluminum oxide and silicon dioxide. An implantable sensor having a rigid substrate may have a sharp point and/or a sharp edge to aid in implantation of a sensor without an additional insertion device.

It will be appreciated that for many sensors and sensor applications, both rigid and flexible sensors will operate adequately. The flexibility of the sensor may also be controlled and varied along a continuum by changing, for example, the composition and/or thickness of the substrate.

In addition to considerations regarding flexibility, it is often desirable that implantable sensors should have a substrate that is physiologically harmless, for example, a substrate approved by a regulatory agency or private institution for in vivo use.

The sensor may include optional features to facilitate insertion of an implantable sensor. For example, the sensor may be pointed at the tip to ease insertion (see FIGS. 5C and 5E). In addition, the sensor may include a barb which assists in anchoring the sensor within the tissue of the user during operation of the sensor. However, the barb is typically small enough so that little damage is caused to the subcutaneous tissue when the sensor is removed for replacement.

An implantable sensor may also, optionally, have an anticlotting agent disposed on a portion of the substrate which is implanted into a user. This anticlotting agent may reduce or eliminate the clotting of blood or other body fluid around the sensor, particularly after insertion of the sensor. Blood clots may foul the sensor or irreproducibly reduce the amount of analyte which diffuses into the sensor. Examples of useful anticlotting agents include heparin and tissue plasminogen activator (TPA), as well as other known anticlotting agents.

The anticlotting agent may be applied to at least a portion of that part of the sensor that is to be implanted. The anticlotting agent may be applied, for example, by bath, spraying, brushing, or dipping, and the like. The anticlotting agent is allowed to dry on the sensor. The anticlotting agent may be immobilized on the surface of the sensor or it may be allowed to diffuse away from the sensor surface. The quantities of anticlotting agent disposed on the sensor may be below the amounts typically used for treatment of medical conditions involving blood clots and, therefore, have only a limited, localized effect.

FIG. 21 shows an example in vivo-based analyte monitoring system 1100 in accordance with certain embodiments of the present disclosure. As shown, analyte monitoring system 1100 includes on body electronics 1110 electrically coupled to in vivo analyte sensor 1101 (a proximal portion of which is shown in FIG. 21) and attached to adhesive layer 1140 for attachment on a skin surface on the body of a user. On body electronics 1110 includes on body housing 1119 that defines an interior compartment. Also shown in FIG. 21 is insertion device 1150 that, when operated, transcutaneously positions a portion of analyte sensor 1101 through a skin surface and in fluid contact with bodily fluid, and positions on body electronics 1110 and adhesive layer 1140 on a skin surface. In some embodiments, on body electronics 1110, analyte sensor 1101 and adhesive layer 1140 are sealed within the housing of insertion device 1150 before use, and in some embodiments, adhesive layer 1140 is also sealed within the housing or itself provides a terminal seal of the insertion device 1150.

With continued reference to FIG. 21, analyte monitoring system 1100 includes display device 1120 (*e.g.,* such as the computing device described herein) which includes a display 1122 to output information to the user, an input component 1121 such as a button, actuator, a touch sensitive switch, a capacitive switch, pressure sensitive switch, jog wheel or the like, to input data or command to display device 1120 or otherwise control the operation of display device 1120. It is noted that some embodiments may include display-less devices or devices without any user interface components. These devices may be functionalized to store data as a data logger and/or provide a conduit to transfer data from on body electronics and/or a display-less device to another device and/or location. Embodiments will be described herein as display devices for example purposes which are in no way intended to limit the embodiments of the present disclosure. It will be apparent that display-less devices may also be used in some embodiments.

In some embodiments, on body electronics 1110 may be configured to store some or all of the monitored analyte related data received from analyte sensor 1101 in a memory during the monitoring time period, and maintain it in memory until the usage period ends. In such embodiments, stored data is retrieved from on body electronics 1110 at the conclusion of the monitoring time period, for example, after removing analyte sensor 1101 from the user by detaching on body electronics 1110 from the skin surface where it was positioned during the monitoring time period. In such data logging configurations, real time monitored analyte level is not communicated to display device 1120 during the monitoring period or otherwise transmitted from on body electronics 1110, but rather, retrieved from on body electronics 1110 after the monitoring time period.

In some embodiments, input component 1121 of display device 1120 may include a microphone and display device 1120 may include software configured to analyze audio input received from the microphone, such that functions and operation of the display device 1120 may be controlled by voice commands. In some embodiments, an output component of display device 1120 includes a speaker for outputting information as audible signals. Similar voice responsive components such as a speaker, microphone and software routines to generate, process and store voice driven signals may be provided to on body electronics 1110.

In some embodiments, display 1122 and input component 1121 may be integrated into a single component, for example a display that can detect the presence and location of a physical contact touch upon the display such as a touch screen user interface. In such embodiments, the user may control the operation of display device 1120 by utilizing a set of pre-programmed motion commands, including, but not limited to, single or double tapping the display, dragging a finger or instrument across the display, motioning multiple fingers or instruments toward one another, motioning multiple fingers or instruments away from one another, and the like. In some embodiments, a display includes a touch screen having areas of pixels with single or dual function capacitive elements that serve as LCD elements and touch sensors.

Display device 1120 also includes data communication port 1123 for wired data communication with external devices such as remote terminal (personal computer) 1170, for example. Example embodiments of the data communication port 1123 include USB port, mini USB port, RS-232 port, Ethernet port, Firewire port, or other similar data communication ports configured to connect to the compatible data cables. Display device 1120 may also include an integrated in vitro glucose meter, including in vitro test strip port 1124 to receive an in vitro glucose test strip for performing in vitro blood glucose measurements.

Referring still to FIG. 21, display 1122 in some embodiments is configured to display a variety of information - some or all of which may be displayed at the same or different time on display 1122. In some embodiments, the displayed information is user-selectable so that a user can customize the information shown on a given display screen. Display 1122 may include, but is not limited to, graphical display 1138, for example, providing a graphical output of glucose values over a monitored time period (which may show important markers such as meals, exercise, sleep, heart rate, blood pressure, and the like), numerical display 1132, for example, providing monitored glucose values (acquired or received in response to the request for the information), and trend or directional arrow display 1131 that indicates a rate of analyte change and/or a rate of the rate of analyte change.

As further shown in FIG. 21, display 1122 may also include date display 1135 providing for example, date information for the user, time of day information display 1139 providing time of day information to the user, battery level indicator display 1133 which graphically shows the condition of the battery (rechargeable or disposable) of the display device 1120, sensor calibration status icon display 1134 for example, in monitoring systems that require periodic, routine or a predetermined number of user calibration events, notifying the user that the analyte sensor calibration is necessary, audio/vibratory settings icon display 1136 for displaying the status of the audio/vibratory output or alarm state, and wireless connectivity status icon display 1137 that provides indication of wireless communication connection with other devices such as on body electronics, data processing module 1160, and/or remote terminal 1170. As additionally shown in FIG. 21, display 1122 may further include simulated touch screen buttons 1140, 1141 for accessing menus, changing display graph output configurations or otherwise for controlling the operation of display device 1120.

Referring back to FIG. 21, in some embodiments, display 1122 of display device 1120 may be additionally, or instead of visual display, configured to output alarms notifications such as alarm and/or alert notifications, glucose values etc., which may be audible, tactile, or any combination thereof. In one aspect, the display device 1120 may include other output components such as a speaker, vibratory output component and the like to provide audible and/or vibratory output indication to the user in addition to the visual output indication provided on display 1122.

After the positioning of on body electronics 1110 on the skin surface and analyte sensor 1101 in vivo to establish fluid contact with interstitial fluid (or other appropriate bodily fluid), on body electronics 1110 in some embodiments is configured to wirelessly communicate analyte related data (such as, for example, data corresponding to monitored analyte level and/or monitored temperature data, and/or stored historical analyte related data) when on body electronics 1110 receives a command or request signal from display device 1120. In some embodiments, on body electronics 1110 may be configured to at least periodically broadcast real time data associated with monitored analyte level which is received by display device 1120 when display device 1120 is within communication range of the data broadcast from on body electronics 1110, *e.g.,* it does not need a command or request from a display device to send information.

For example, display device 1120 may be configured to transmit one or more commands to on body electronics 1110 to initiate data transfer, and in response, on body electronics 1110 may be configured to wirelessly transmit stored analyte related data collected during the monitoring time period to display device 1120. Display device 1120 may in turn be connected to a remote terminal 1170 such as a personal computer and functions as a data conduit to transfer the stored analyte level information from the on body electronics 1110 to remote terminal 1170. In some embodiments, the received data from the on body electronics 1110 may be stored (permanently or temporarily) in one or more memory of the display device 1120. In certain other embodiments, display device 1120 is configured as a data conduit to pass the data received from on body electronics 1110 to remote terminal 1170 that is connected to display device 1120.

Referring still to FIG. 21, also shown in analyte monitoring system 1100 are data processing module 1160 and remote terminal 1170. Remote terminal 1170 may include a personal computer, a server terminal a laptop computer or other suitable data processing devices including software for data management and analysis and communication with the components in the analyte monitoring system 1100. For example, remote terminal 1170 may be connected to a local area network (LAN), a wide area network (WAN), or other data network for uni-directional or bi-directional data communication between remote terminal 1170 and display device 1120 and/or data processing module 1160.

Remote terminal 1170 in some embodiments may include one or more computer terminals located at a physician's office or a hospital. For example, remote terminal 1170 may be located at a location other than the location of display device 1120. Remote terminal 1170 and display device 1120 could be in different rooms or different buildings. Remote terminal 1170 and display device 1120 could be at least about one mile apart, *e.g.,* at least about 10 miles apart, *e.g.,* at least about 1100 miles apart. For example, remote terminal 1170 could be in the same city as display device 1120, remote terminal 1170 could be in a different city than display device 1120, remote terminal 1170 could be in the same state as display device 1120, remote terminal 1170 could be in a different state than display device 1120, remote terminal 1170 could be in the same country as display device 1120, or remote terminal 1170 could be in a different country than display device 1120, for example.

In some embodiments, a separate, optional data communication/processing device such as data processing module 1160 may be provided in analyte monitoring system 1100. Data processing module 1160 may include components to communicate using one or more wireless communication protocols such as, for example, but not limited to, infrared (IR) protocol, Bluetooth protocol, Zigbee protocol, and 802.11 wireless LAN protocol. Additional description of communication protocols including those based on Bluetooth protocol and/or Zigbee protocol can be found in U.S. Patent Publication No. 2006/0193375 incorporated herein by reference in its entirety for all purposes. Data processing module 1160 may further include communication ports, drivers or connectors to establish wired communication with one or more of display device 1120, on body electronics 1110, or remote terminal 1170 including, for example, but not limited to USB connector and/or USB port, Ethernet connector and/or port, FireWire connector and/or port, or RS-232 port and/or connector.

In some embodiments, data processing module 1160 is programmed to transmit a polling or query signal to on body electronics 1110 at a predetermined time interval (*e.g.,* once every minute, once every five minutes, or the like), and in response, receive the monitored analyte level information from on body electronics 1110. Data processing module 1160 stores in its memory the received analyte level information, and/or relays or retransmits the received information to another device such as display device 1120. More specifically in some embodiments, data processing module 1160 may be configured as a data relay device to retransmit or pass through the received analyte level data from on body electronics 1110 to display device 1120 or a remote terminal (for example, over a data network such as a cellular or WiFi data network) or both.

In some embodiments, on body electronics 1110 and data processing module 1160 may be positioned on the skin surface of the user within a predetermined distance of each other (for example, about 1-12 inches, or about 1-10 inches, or about 1-7 inches, or about 1-5 inches) such that periodic communication between on body electronics 1110 and data processing module 1160 is maintained. Alternatively, data processing module 1160 may be worn on a belt or clothing item of the user, such that the desired distance for communication between the on body electronics 1110 and data processing module 1160 for data communication is maintained. In a further aspect, the housing of data processing module 1160 may be configured to couple to or engage with on body electronics 1110 such that the two devices are combined or integrated as a single assembly and positioned on the skin surface. In further embodiments, data processing module 1160 is detachably engaged or connected to on body electronics 1110 providing additional modularity such that data processing module 1160 may be optionally removed or reattached as desired.

Referring again to FIG. 21, in some embodiments, data processing module 1160 is programmed to transmit a command or signal to on body electronics 1110 at a predetermined time interval such as once every minute, or once every 5 minutes or once every 30 minutes or any other suitable or desired programmable time interval to request analyte related data from on body electronics 1110. When data processing module 1160 receives the requested analyte related data, it stores the received data. In this manner, analyte monitoring system 1100 may be configured to receive the continuously monitored analyte related information at the programmed or programmable time interval, which is stored and/or displayed to the user. The stored data in data processing module 1160 may be subsequently provided or transmitted to display device 1120, remote terminal 1170 or the like for subsequent data analysis such as identifying frequency of periods of glycemic level excursions over the monitored time period, or the frequency of the alarm event occurrence during the monitored time period, for example, to improve therapy related decisions. Using this information, the doctor, healthcare provider or the user may adjust or recommend modification to the diet, daily habits and routines such as exercise, and the like.

In another embodiment, data processing module 1160 transmits a command or signal to on body electronics 1110 to receive the analyte related data in response to a user activation of a switch provided on data processing module 1160 or a user initiated command received from display device 1120. In further embodiments, data processing module 1160 is configured to transmit a command or signal to on body electronics 1110 in response to receiving a user initiated command only after a predetermined time interval has elapsed. For example, in some embodiments, if the user does not initiate communication within a programmed time period, such as, for example about 5 hours from last communication (or 10 hours from the last communication, or 24 hours from the last communication), the data processing module 1160 may be programmed to automatically transmit a request command or signal to on body electronics 1110. Alternatively, data processing module 1160 may be programmed to activate an alarm to notify the user that a predetermined time period of time has elapsed since the last communication between the data processing module 1160 and on body electronics 1110. In this manner, users or healthcare providers may program or configure data processing module 1160 to provide certain compliance with analyte monitoring regimen, so that frequent determination of analyte levels is maintained or performed by the user.

In some embodiments, when a programmed or programmable alarm condition is detected (for example, a detected glucose level monitored by analyte sensor 1101 that is outside a predetermined acceptable range indicating a physiological condition which requires attention or intervention for medical treatment or analysis (for example, a hypoglycemic condition, a hyperglycemic condition, an impending hyperglycemic condition or an impending hypoglycemic condition), the one or more output indications may be generated by the control logic or processor of the on body electronics 1110 and output to the user on a user interface of on body electronics 1110 so that corrective action may be timely taken. In addition to or alternatively, if display device 1120 is within communication range, the output indications or alarm data may be communicated to display device 1120 whose processor, upon detection of the alarm data reception, controls the display 1122 to output one or more notification.

In some embodiments, control logic or processors of on body electronics 1110 can execute software programs stored in memory to determine future or anticipated analyte levels based on information obtained from analyte sensor 1101, *e.g.,* the current analyte level, the rate of change of the analyte level, the acceleration of the analyte level change, and/or analyte trend information determined based on stored monitored analyte data providing a historical trend or direction of analyte level fluctuation as function time during monitored time period. Predictive alarm parameters may be programmed or programmable in display device 1120, or the on body electronics 1110, or both, and output to the user in advance of anticipating the user's analyte level reaching the future level. This provides the user an opportunity to take timely corrective action.

Information, such as variation or fluctuation of the monitored analyte level as a function of time over the monitored time period providing analyte trend information, for example, may be determined by one or more control logic or processors of display device 1120, data processing module 1160, and/or remote terminal 1170, and/or on body electronics 1110. Such information may be displayed as, for example, a graph (such as a line graph) to indicate to the user the current and/or historical and/or and predicted future analyte levels as measured and predicted by the analyte monitoring system 1100. Such information may also be displayed as directional arrows (for example, see trend or directional arrow display 1131) or other icon(s), *e.g.,* the position of which on the screen relative to a reference point indicated whether the analyte level is increasing or decreasing as well as the acceleration or deceleration of the increase or decrease in analyte level. This information may be utilized by the user to determine any necessary corrective actions to ensure the analyte level remains within an acceptable and/or clinically safe range. Other visual indicators, including colors, flashing, fading, etc., as well as audio indicators including a change in pitch, volume, or tone of an audio output and/or vibratory or other tactile indicators may also be incorporated into the display of trend data as means of notifying the user of the current level and/or direction and/or rate of change of the monitored analyte level. For example, based on a determined rate of glucose change, programmed clinically significant glucose threshold levels (*e.g.,* hyperglycemic and/or hypoglycemic levels), and current analyte level derived by an in vivo analyte sensor, the system 1100 may include an algorithm stored on computer readable medium to determine the time it will take to reach a clinically significant level and will output notification in advance of reaching the clinically significant level, *e.g.,* 30 minutes before a clinically significant level is anticipated, and/or 20 minutes, and/or 10 minutes, and/or 5 minutes, and/or 3 minutes, and/or 1 minute, and so on, with outputs increasing in intensity or the like.

Referring again back to FIG. 21, in some embodiments, software algorithm(s) for execution by data processing module 1160 may be stored in an external memory device such as an SD card, microSD card, compact flash card, XD card, Memory Stick card, Memory Stick Duo card, or USB memory stick/device including executable programs stored in such devices for execution upon connection to the respective one or more of the on body electronics 1110, remote terminal 1170 or display device 1120. In a further aspect, software algorithms for execution by data processing module 1160 may be provided to a communication device such as a mobile telephone including, for example, WiFi or Internet enabled smart phones or personal digital assistants (PDAs) as a downloadable application for execution by the downloading communication device.

Examples of smart phones include Windows^{®}, Android^{™}, iPhone^{®} operating system, Palm^{®} WebOS^{™}, Blackberry^{®} operating system, or Symbian^{®} operating system based mobile telephones with data network connectivity functionality for data communication over an internet connection and/or a local area network (LAN). PDAs as described above include, for example, portable electronic devices including one or more processors and data communication capability with a user interface (*e.g.,* display/output unit and/or input unit, and configured for performing data processing, data upload/download over the internet, for example. In such embodiments, remote terminal 1170 may be configured to provide the executable application software to the one or more of the communication devices described above when communication between the remote terminal 1170 and the devices are established.

In still further embodiments, executable software applications may be provided over-the-air (OTA) as an OTA download such that wired connection to remote terminal 1170 is not necessary. For example, executable applications may be automatically downloaded as software download to the communication device, and depending upon the configuration of the communication device, installed on the device for use automatically, or based on user confirmation or acknowledgement on the communication device to execute the installation of the application. The OTA download and installation of software may include software applications and/or routines that are updates or upgrades to the existing functions or features of data processing module 1160 and/or display device 1120.

Referring back to remote terminal 1170 of FIG. 21, in some embodiments, new software and/or software updates such as software patches or fixes, firmware updates or software driver upgrades, among others, for display device 1120 and/or on body electronics 1110 and/or data processing module 1160 may be provided by remote terminal 1170 when communication between the remote terminal 1170 and display device 1120 and/or data processing module 1160 is established. For example, software upgrades, executable programming changes or modification for on body electronics 1110 may be received from remote terminal 1170 by one or more of display device 1120 or data processing module 1160, and thereafter, provided to on body electronics 1110 to update its software or programmable functions. For example, in some embodiments, software received and installed in on body electronics 1110 may include software bug fixes, modification to the previously stalled software parameters (modification to analyte related data storage time interval, resetting or adjusting time base or information of on body electronics 1110, modification to the transmitted data type, data transmission sequence, or data storage time period, among others).

### On Body Electronics

In some embodiments, on body electronics (or sensor control device) 1110 (FIG. 21) includes at least a portion of the electronic components that operate the sensor and the display device. The electronic components of the on body electronics typically include a power supply for operating the on body electronics and the sensor, a sensor circuit for obtaining signals from and operating the sensor, a measurement circuit that converts sensor signals to a desired format, and a processing circuit (or processing circuitry) that, at minimum, obtains signals from the sensor circuit and/or measurement circuit and provides the signals to an optional on body electronics. In some embodiments, the processing circuit may also partially or completely evaluate the signals from the sensor and convey the resulting data to the optional on body electronics and/or activate an optional alarm system if the analyte level exceeds a threshold. The processing circuit often includes digital logic circuitry.

The on body electronics may optionally contain electronics for transmitting the sensor signals or processed data from the processing circuit to a receiver/display unit; a data storage unit for temporarily or permanently storing data from the processing circuit; a temperature probe circuit for receiving signals from and operating a temperature probe; a reference voltage generator for providing a reference voltage for comparison with sensor-generated signals; and/or a watchdog circuit that monitors the operation of the electronic components in the on body electronics.

Moreover, the on body electronics may also include digital and/or analog components utilizing semiconductor devices, including transistors. To operate these semiconductor devices, the on body electronics may include other components including, for example, a bias control generator to correctly bias analog and digital semiconductor devices, an oscillator to provide a clock signal, and a digital logic and timing component to provide timing signals and logic operations for the digital components of the circuit.

As an example of the operation of these components, the sensor circuit and the optional temperature probe circuit provide raw signals from the sensor to the measurement circuit. The measurement circuit converts the raw signals to a desired format, using for example, a current-to-voltage converter, current-to-frequency converter, and/or a binary counter or other indicator that produces a signal proportional to the absolute value of the raw signal. This may be used, for example, to convert the raw signal to a format that can be used by digital logic circuits. The processing circuit may then, optionally, evaluate the data and provide commands to operate the electronics.

Referring to FIG, 21, in some embodiments, adhesive patch 1140 has an on body footprint that is less than about 3.0 inches in diameter, *e.g.,* less than about 2.0 inches in diameter, less than about 1.0 inches in diameter, where in some embodiments an adhesive patch may have a diameter that is 1.0 inch to about 1.5 inches or less.

In some embodiments, on body electronics 1110 is configured such that it has a small surface area, *e.g.,* less than about 2 square inches excluding adhesive patch 1140, *e.g.,* less than about 1.5 square inches excluding adhesive patch 1140, *e.g.,* less than about 1 square inches excluding adhesive patch 1140, *e.g.,* less than about 0.9 square inches excluding adhesive patch 1140, *e.g.,* less than about 0.8 square inches excluding adhesive patch 1140, *e.g.,* less than about 0.75 square inches excluding adhesive patch 1140, *e.g.,* less than about .7 square inches excluding adhesive patch 1140, where in some embodiments the surface area of an on body electronics unit may be about 0.75 square inches to about 0.79 square inches excluding an adhesive patch 1140.

In some embodiments, on body electronics 1110, including adhesive patch 1140, has a surface area that is about 3.0 square inches or less including an adhesive patch, *e.g.,* about 2.0 square inches or less including an adhesive patch, *e.g.,* about 1.9 square inches or less including an adhesive patch, *e.g.,* about 1.8 square inches or less including an adhesive patch, *e.g.,* about 1.75 square inches or less including an adhesive patch, *e.g.,* about 1.6 square inches or less including an adhesive patch, where in some embodiments the surface area of an on body electronics unit may be about 1.75 square inches to about 1.77 square inches or less.

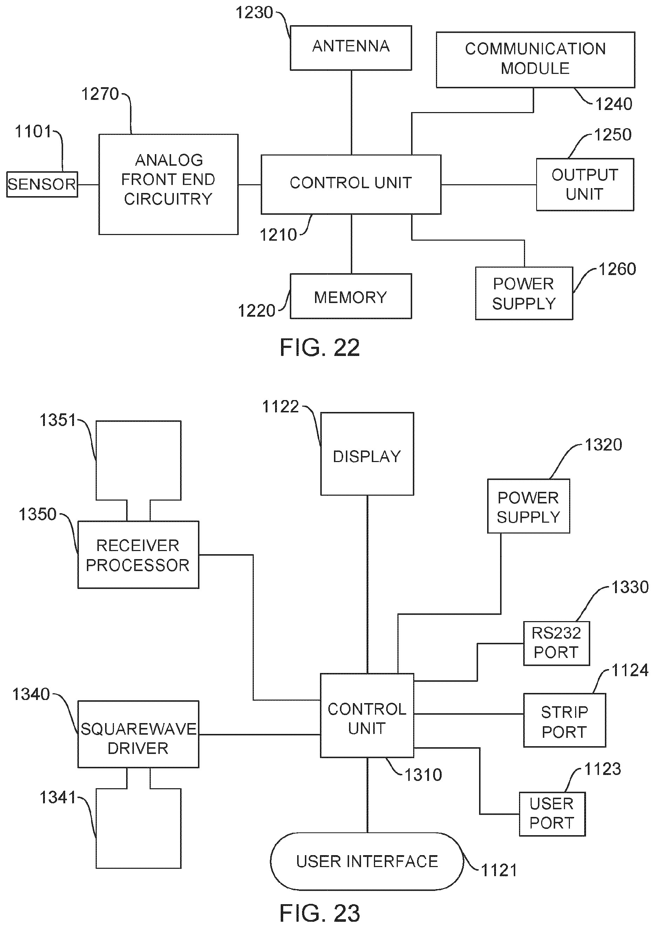

FIG. 22 is a block diagram of the on body electronics 1110 (FIG. 21) in some embodiments. Referring to FIG. 22, on body electronics 1110 in some embodiments includes a control unit 1210 (such as, for example but not limited to, one or more processors (or processing circuitry) and/or ASICs with processing circuitry), operatively coupled to analog front end circuitry 1270 to process signals such as raw current signals received from analyte sensor 1101. Also shown in FIG. 22 is memory 1220 operatively coupled to control unit 1210 for storing data and/or software routines for execution by control unit 1210. Memory 1220 in some embodiments may include electrically erasable programmable read only memory (EEPROM), erasable programmable read only memory (EPROM), random access memory (RAM), read only memory (ROM), flash memory, or one or more combinations thereof.

In some embodiments, control unit 1210 accesses data or software routines stored in the memory 1220 to update, store or replace stored data or information in the memory 1220, in addition to retrieving one or more stored software routines for execution. Also shown in FIG. 22 is power supply 1260 which, in some embodiments, provides power to some or all of the components of on body electronics 1110. For example, in some embodiments, power supply 1260 is configured to provide power to the components of on body electronics 1110 except for communication module 1240. In such embodiments, on body electronics 1110 is configured to operate analyte sensor 1101 to detect and monitor the analyte level at a predetermined or programmed (or programmable) time intervals, and generating and storing, for example, the signals or data corresponding to the detected analyte levels.

In some embodiments, power supply 1260 in on body electronics 1110 may be toggled between its internal power source (*e.g.,* a battery) and the RF power received from display device 1120. For example, in some embodiments, on body electronics 1110 may include a diode or a switch that is provided in the internal power source connection path in on body electronics 1110 such that, when a predetermined level of RF power is detected by on body electronics 1110, the diode or switch is triggered to disable the internal power source connection (*e.g.,* making an open circuit at the power source connection path), and the components of on body electronics is powered with the received RF power. The open circuit at the power source connection path prevents the internal power source from draining or dissipating as in the case when it is used to power on body electronics 1110.

When the RF power from display device 1120 falls below the predetermined level, the diode or switch is triggered to establish the connection between the internal power source and the other components of on body electronics 1110 to power the on body electronics 1110 with the internal power source. In this manner, in some embodiments, toggling between the internal power source and the RF power from display device 1120 may be configured to prolong or extend the useful life of the internal power source.

The stored analyte related data, however, is not transmitted or otherwise communicated to another device such as display device 1120 (FIG. 21) until communication module 1240 is separately powered, for example, with the RF power from display device 1120 that is positioned within a predetermined distance from on body electronics 1110. In such embodiments, analyte level is sampled based on the predetermined or programmed time intervals as discussed above, and stored in memory 1220. When analyte level information is requested, for example, based on a request or transmit command received from another device such as display device 1120 (FIG. 21), using the RF power from the display device, communication module 1240 of on body electronics 1110 initiates data transfer to the display device 1120.

Referring back to FIG. 22, an optional output unit 1250 is provided to on body electronics 1110. In some embodiments, output unit 1250 may include an LED indicator, for example, to alert the user of one or more predetermined conditions associated with the operation of the on body electronics 1110 and/or the determined analyte level. By way of non-limiting example, the on body electronics 1110 may be programmed to assert a notification using an LED indicator, or other indicator on the on body electronics 1110 when signals (based on one sampled sensor data point, or multiple sensor data points) received from analyte sensor 1101 are indicated to be beyond a programmed acceptable range, potentially indicating a health risk condition such as hyperglycemia or hypoglycemia, or the onset or potential of such conditions. With such prompt or indication, the user may be timely informed of such potential condition, and using display device 1120, acquire the glucose level information from the on body electronics 1110 to confirm the presence of such conditions so that timely corrective actions may be taken.

Referring again to FIG. 22, antenna 1230 and communication module 1240 operatively coupled to the control unit 1210 may be configured to detect and process the RF power when on body electronics 1110 is positioned within predetermined proximity to the display device 1120 (FIG. 21) that is providing or radiating the RF power. Further, on body electronics 1110 may provide analyte level information and optionally analyte trend or historical information based on stored analyte level data, to display device 1120. In certain aspects, the trend information may include a plurality of analyte level information over a predetermined time period that are stored in the memory 1220 of the on body electronics 1110 and provided to the display device 1120 with the real time analyte level information. For example, the trend information may include a series of time spaced analyte level data for the time period since the last transmission of the analyte level information to the display device 1120. Alternatively, the trend information may include analyte level data for the prior 30 minutes or one hour that are stored in memory 1220 and retrieved under the control of the control unit 1210 for transmission to the display device 1120.

In some embodiments, on body electronics 1110 is configured to store analyte level data in first and second FIFO buffers that are part of memory 1220. The first FIFO buffer stores 16 (or 10 or 20) of the most recent analyte level data spaced one minute apart. The second FIFO buffer stores the most recent 8 hours (or 10 hours or 3 hours) of analyte level data spaced 10 minutes (or 15 minutes or 20 minutes). The stored analyte level data are transmitted from on body electronics 1110 to display unit 1120 in response to a request received from display unit 1120. Display unit 1120 uses the analyte level data from the first FIFO buffer to estimate glucose rate-of-change and analyte level data from the second FIFO buffer to determine historical plots or trend information.

In some embodiments, for configurations of the on body electronics that includes a power supply, the on body electronics may be configured to detect an RF control command (ping signal) from the display device 1120. More specifically, an On/Off Key (OOK) detector may be provided in the on body electronics which is turned on and powered by the power supply of the on body electronics to detect the RF control command or the ping signal from the display device 1120. Additional details of the OOK detector are provided in U.S. Patent Publication No. 2008/0278333, the disclosure of which is incorporated by reference in its entirety for all purposes. In certain aspects, when the RF control command is detected, on body electronics determines what response packet is necessary, and generates the response packet for transmission back to the display device 1120. In this embodiment, the analyte sensor 1101 continuously receives power from the power supply or the battery of the on body electronics and operates to monitor the analyte level continuously in use. However, the sampled signal from the analyte sensor 1101 may not be provided to the display device 1120 until the on body electronics receives the RF power (from the display device 1120) to initiate the transmission of the data to the display device 1120. In one embodiment, the power supply of the on body electronics may include a rechargeable battery which charges when the on body electronics receives the RF power (from the display device 1120, for example).

Referring back to FIG. 21, in some embodiments, on body electronics 1110 and the display device 1120 may be configured to communicate using RFID (radio frequency identification) protocols. More particularly, in some embodiments, the display device 1120 is configured to interrogate the on body electronics 1110 (associated with an RFID tag) over an RF communication link, and in response to the RF interrogation signal from the display device 1120, on body electronics 1110 provides an RF response signal including, for example, data associated with the sampled analyte level from the sensor 1101. Additional information regarding the operation of RFID communication can be found in U.S. Patent No. 7,545,272, and in US Application Nos. 12/698,624, 12/699,653, 12/761,387, and U.S. Patent Publication No. 2009/0108992, the disclosures of all of which are incorporated herein by reference in their entireties and for all purposes.

For example, in one embodiment, the display device 1120 may include a backscatter RFID reader configured to provide an RF field such that when on body electronics 1110 is within the transmitted RF field of the RFID reader, on body electronics 1110 antenna is tuned and in turn provides a reflected or response signal (for example, a backscatter signal) to the display device 1120. The reflected or response signal may include sampled analyte level data from the analyte sensor 1101.

In some embodiments, when display device 1120 is positioned in within a predetermined range of the on body electronics 1110 and receives the response signal from the on body electronics 1110, the display device 1120 is configured to output an indication (audible, visual or otherwise) to confirm the analyte level measurement acquisition. That is, during the course of the 5 to 10 days of wearing the on body electronics 1110, the user may at any time position the display device 1120 within a predetermined distance (for example, about 1-5 inches, or about 1-10 inches, or about 1-12 inches) from on body electronics 1110, and after waiting a few seconds of sample acquisition time period, an audible indication is output confirming the receipt of the real time analyte level information. The received analyte information may be output to the display 1122 (FIG. 21) of the display device 1120 for presentation to the user.

In some embodiments, on body electronics 1110 includes an ASIC that includes on chip a RISC (reduced instruction set computing) processor, an EEPROM, and a register (A/D converter operatively coupled to an analyte sensor). EEPROM in some embodiments includes a portion that has programmed in it one or more characteristics or details associated with a memory management routine. Example characteristics or details include, for example, a source address (*e.g.,* whether it is an array or a single memory location), a destination address, a size/number of bytes to copy to memory, whether the memory location is a loop buffer (*e.g.,* overwriting the older stored values with new values when the end of the buffer is reached).

In some embodiments, a preset number of specific events may be fined and stored. For example, such events may include, but not limited to (1) RF power on event, (2) RF data read command; (3) RF data log command, (4) one minute data ready event (*e.g.,* the A/D conversion of the signal from the analyte sensor is complete and the digitized data is ready for storage), or (3) log data (10 minute analyte data) ready event (*e.g.,* when 10 minutes of analyte data is available for storage). For example, 10 minutes of analyte data is available in some embodiments when the last A/D conversion for the 10 minute analyte data is complete. In some embodiments, other events or states may be defined.

In some embodiments, when the RISC processor detects one of the specific events, the RISC processor executes the programmed memory management routine. During the execution of the memory management routine, the stored characteristics in EEPROM are retrieved. Based on the retrieved characteristics, the memory management routine stores data associated with the detected event. For example, in some embodiments, when a RF data log command event is detected, the data associated with this event is logged in another section of the EEPROM on ASIC chip in accordance with the retrieved characteristics (*e.g.,* source and destination address for the data associated with this event).

In some embodiments, the characteristics stored in EEPROM associated with the specific events may be modified. For example, the source and destination address may be changed or modified to point to a different memory device or storage unit of on body electronics 1110 (*e.g.,* a separate EEPROM or memory that is not part of the ASIC chip). For example, data logger applications of the monitoring system 1100 requires storing an amount of data (*e.g.,* data for about 30 days, about 45 days, about 60 days or more, of 1 minute interval sampled analyte data (or 5 minute interval sampled data, or 10 minute interval sampled data)) in on body electronics 1110 much greater than in on demand application where a limited amount of data is stored (*e.g.,* 15 samples of 1 minute interval sampled analyte data, and 6 hours of historical 10 minute interval sampled analyte data). In some embodiments, the amount of data for storage in data logger application may exceed the capacity of on chip EEPROM. In such cases, a larger capacity, off chip EEPROM may be provided in on body electronics 1110 for storing data from the data logger application. To configure on body electronics 1110 to store sampled analyte data in the larger capacity, off chip EEPROM, in some embodiments, the characteristics stored in EEPROM associated with the events are reprogrammed or updated (for example, by updating the source and destination addresses associated with the events) so that data logging or storage is pointed to the larger off chip EEPROM.

In this manner, by updating or reprogramming the portion of on chip EEPROM that stores the event characteristics, location of data storage in on body electronics 1110 may be updated or modified depending upon the desired application or use of on body electronics 1110. Furthermore, other stored characteristics associated with one or more particular events may be updated or reprogrammed in EEPROM as desired to modify the use or application of on body electronics 1110 in analyte monitoring system 1100. This is further advantageously achieved without reprogramming or modifying the stored routines for executing the particular events by the RISC processor.

### Display Devices/Computing Devices

FIG. 23 is a block diagram of display device 1120 as shown in FIG. 21 in some embodiments. Although the term display device is used, the device can be configured to read without displaying data, and can be provided without a display, such as can be the case with a relay or other device that relays a received signal according to the same or a different transmission protocol (*e.g.,* NFC-to-Bluetooth or Bluetooth Low Energy). Referring to FIG. 23, display device 1120 (FIG. 21) includes control unit 1310, such as one or more processors (or processing circuitry) operatively coupled to a display 1122, and an input component (*e.g.,* user interface) 1121. The display device 1120 may also include one or more data communication ports such as USB port (or connector) 1123 or RS-232 port 1330 (or any other wired communication ports) for data communication with a data processing module 1160 (FIG. 21), remote terminal 1170 (FIG. 21), or other devices such as a personal computer, a server, a mobile computing device, a mobile telephone, a pager, or other handheld data processing devices including mobile telephones such as internet connectivity enabled smart phones, with data communication and processing capabilities including data storage and output.

Referring back to FIG. 23, display device 1120 may include a strip port 1124 configured to receive in vitro test strips, the strip port 1124 coupled to the control unit 1310, and further, where the control unit 1310 includes programming to process the sample on the in vitro test strip which is received in the strip port 1124. Any suitable in vitro test strip may be employed, e.g., test strips that only require a very small amount (*e.g.,* one microliter or less, *e.g.,* about 0.5 microliter or less, *e.g.,* about 0.1 microliter or less), of applied sample to the strip in order to obtain accurate glucose information. Display devices with integrated in vitro monitors and test strip ports may be configured to conduct in vitro analyte monitoring with no user calibration of the in vitro test strips (*e.g.,* no human intervention calibration).

In some embodiments, an integrated in vitro meter can accept and process a variety of different types of test strips (*e.g.,* those that require user calibration and those that do not), some of which may use different technologies (*e.g.,* those that operate using amperometric techniques and those that operate using coulometric techniques, and the like). Detailed description of such test strips and devices for conducting in vitro analyte monitoring is provided in U.S. Patent Nos. 6,377,894, 6,616,819, 7,749,740, 7,418,285; U.S. Patent Publication Nos. 2004/0118704, 2006/0096006, 2008/0066305, 2008/0267823, 2010/0094610, 2010/0094111, and 2010/0094112, and U.S. Application No. 12/695,947, the disclosures of all of which are incorporated herein by reference in their entireties and for all purposes.

Glucose information obtained by the in vitro glucose testing device may be used for a variety of purposes. For example, the information may be used to calibrate analyte sensor 1101 (FIG. 21) if the sensor requires in vivo calibration, confirm results of analyte sensor 1101 to increase the confidence in the results from sensor 1101 indicating the monitored analyte level (*e.g.,* in instances in which information obtained by sensor 1101 is employed in therapy related decisions), etc. In some embodiments, analyte sensors do not require calibration by human intervention during its usage life. However, in some embodiments, a system may be programmed to self-detect problems and take action, *e.g.,* shut off and/or notify a user. For example, an analyte monitoring system may be configured to detect system malfunction, or potential degradation of sensor stability or potential adverse condition associated with the operation of the analyte sensor, the system may notify the user, using display device 1120 (FIG. 21) for example, to perform analyte sensor calibration or compare the results received from the analyte sensor corresponding to the monitored analyte level, to a reference value (such as a result from an in vitro blood glucose measurement).

In some embodiments, when the potential adverse condition associated with the operation of the sensor, and/or potential sensor stability degradation condition is detected, the system may be configured to shut down (automatically without notification to the user, or after notifying the user) or disable the output or display of the monitored analyte level information received the on body electronics assembly. In some embodiments, the analyte monitoring system may be shut down or disabled temporarily to provide an opportunity to the user to correct any detected adverse condition or sensor instability. In certain other embodiments, the analyte monitoring system may be permanently disabled when the adverse sensor operation condition or sensor instability is detected.

With continued reference to FIG. 23, power supply 1320, such as one or more batteries, rechargeable or single use disposable, is also provided and operatively coupled to control unit 1310, and configured to provide the necessary power to display device 1120 (FIG. 21) for operation. In addition, display device 1120 may include an antenna 1351 such as a 433MHz (or other equivalent) loop antenna, 13.56 MHz antenna, or a 2.45GHz antenna, coupled to a receiver processor 1350 (which may include a 433MHz, 13.56MHz, or 2.45GHz transceiver chip, for example) for wireless communication with the on body electronics 1110 (FIG. 21). Additionally, an inductive loop antenna 1341 is provided and coupled to a squarewave driver 1340 which is operatively coupled to control unit 1310.

In some embodiments, data packets received from on body electronics and received in response to a request from display device, for example, include one or more of a current glucose level from the analyte sensor, a current estimated rate of glycemic change, and a glucose trend history based on automatic readings acquired and stored in memory of on skin electronics. For example, current glucose level may be output on display 1122 of display device 1120 as a numerical value, the current estimated rage of glycemic change may be output on display 1122 as a directional arrow 1131 (FIG. 21), and glucose trend history based on stored monitored values may be output on display 1122 as a graphical trace 1138 (FIG. 21). In some embodiments, the processor (or processing circuitry) of display device 1120 may be programmed to output more or less information for display on display 1122, and further, the type and amount of information output on display 1122 may be programmed or programmable by the user.

In some embodiments, display device 1120 is programmed to maintain a time period between each consecutive of analyte data request from on body electronics 1110. For example, in some embodiments, display device 1120 is configured such that after an initial analyte data request has been sent to on body electronics 1110, and the monitored analyte level information received from on body electronics 1110, display device 1120 disallows a subsequent analyte data request to be sent to on body electronics 1110 until a predetermined time period has elapsed measured from the transmission of the initial analyte data request. For example, when display device 1120 is operated to send to on body electronics 1110 a request for analyte related data, an internal clock or timer of the display device 1120 starts or activates the internal clock or timer programmed with a predetermined time period to count down. Display device 1120 in some embodiments include programming to disable or prevent sending the second, subsequent request for analyte data from on body electronics 1110 until after the predetermined time period has elapsed.

In some embodiments, the predetermined time period includes about 120 seconds, about 90 seconds, about 60 seconds, or about 30 seconds or less. The predetermined time period in some embodiments is determined by the time period for performing analog to digital conversion by on body electronics 1110 to convert the sampled signal from monitoring the analyte level to a corresponding digital signal for transmission and/or the sampling period of analyte sensor 1101, monitoring analyte level every minute, or every 5 minutes, or every 10 minutes or other suitable time interval. The time interval in some embodiments may be pre-programmed as software logic in on body electronics 1110, or alternatively, is programmable and can be modified during in vivo sensor use.

In some embodiments, display device 1120 may be programmed or programmable to discard or identify received data from on body electronics 1110 that is corrupt or otherwise includes error. For example, in some embodiments, a minimum time period between subsequent analyte data request is not enforced or programmed in display device 1120. However, display device 1120 includes software routines that identify data that is corrupt or not based on examining the data packet. For example, each data packet received from on body electronics 1110 includes a single bit or a byte or other suitable portion of the data packet that provides an indication of the data status. In the case of a single bit as the data status identifier in the data packet from on body electronics 1110, in some embodiments, a value of 1 indicates that the data is not corrupt. In such embodiments, on body electronics 1110 is configured to reset this bit in the data packet to 0 at the end of each sampling period (for example, after each minute), and change the value to 1 when the A/D conversion routine is completed during the sampling period without error.

### Data Communication and Processing Routines

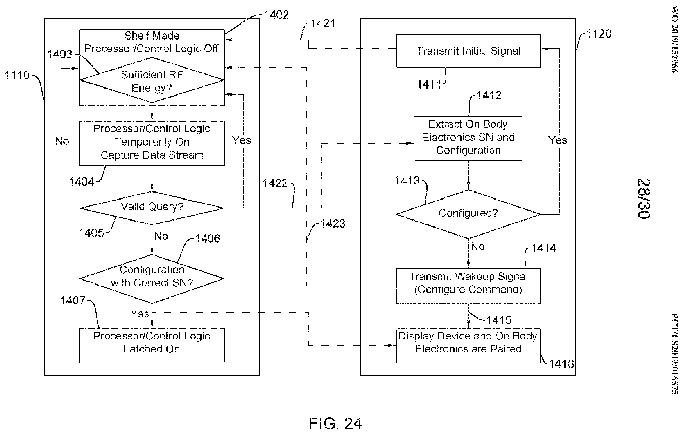

Referring now to FIG. 24 which illustrates data and/or commands exchange between on body electronics 1110 and display device 1120 during the initialization and pairing routine, display device 1120 provides and initial signal 1421 to on body electronics 1110. When the received initial signal 1421 includes RF energy exceeding a predetermined threshold level 1403, an envelope detector of on body electronics 1110 is triggered 1404, one or more oscillators of on body electronics 1110 turns on, and control logic or processors of on body electronics 1110 is temporarily latched on to retrieve and execute one or more software routines to extract the data stream from the envelope detector 1404. If the data stream from the envelope detector returns a valid query 1405, a reply signal 1422 is transmitted to display device 1120. The reply signal 1422 from on body electronics 1110 includes an identification code such as on body electronics 1110 serial number. Thereafter, the on body electronics 1110 returns to shelf mode in an inactive state.

On the other hand, if the data stream from the envelope detector does not return a valid query from display device 1120, on body electronics 1110 does not transmit a reply signal to display device 1120 nor is on body electronics 1110 serial number provided to display device 1120. Thereafter, on body electronics 1110 returns to shelf mode 1403, and remains in powered down state until it detects a subsequent initial signal 1421 from display device 1120.

When display device 1120 receives the data packet including identification information or serial number from on body electronics 1110, it extracts that information from the data packet 1412. With the extracted on body electronics 1110 serial number, display device 1120 determines whether on body electronics 1110 associated with the received serial number is configured. If on body electronics 1110 associated with the received serial number has already been configured, for example, by another display device, display device 1120 returns to the beginning of the routine to transmit another initial signal 1411 in an attempt to initialize another on body electronics that has not been configured yet. In this manner, in some embodiments, display device 1120 is configured to pair with an on body electronics that has not already been paired with or configured by another display device.

Referring back to FIG. 24, if on body electronics 1110 associated with the extracted serial number has not been configured 1413, display device 1120 is configured to transmit a wake up signal to on body electronics 1110 which includes a configure command. In some embodiments, wake up command from display device 1120 includes a serial number of on body electronics 1110 so that only the on body electronics with the same serial number included in the wake up command detects and exits the inactive shelf mode and enters the active mode. More specifically, when the wake up command including the serial number is received by on body electronics 1110, control logic or one or more processors (or processing circuitry) of on body electronics 1110 executes routines 1403, 1404, and 1405 to temporarily exit the shelf mode, when the RF energy received with the wakeup signal (including the configure command) exceeds the threshold level, and determines that it is not a valid query (as that determination was previously made and its serial number transmitted to display device 1120). Thereafter, on body electronics 1110 determines whether the received serial number (which was received with the wake up command) matches its own stored serial number 1406. If the two serial numbers do not match, routine returns to the beginning where on body electronics 1110 is again placed in inactive shelf mode 1402. On the other hand, if on body electronics 1110 determines that the received serial number matches its stored serial number 1406, control logic or one or more processors of on body electronics 1110 permanently latches on 1407, and oscillators are turned on to activate on body electronics 1110. Further, referring back to FIG, 24, when on body electronics 1110 determines that the received serial number matches its own serial number 1406, display device 1120 and on body electronics 1110 are successfully paired 1416.

In this manner, using a wireless signal to turn on and initialize on body electronics 1110, the shelf life of on body electronics 1110 may be prolonged since very little current is drawn or dissipated from on body electronics 1110 power supply during the time period that on body electronics 1110 is in inactive, shelf mode prior to operation. In some embodiments, during the inactive shelf mode, on body electronics 1110 has minimal operation, if any, that require extremely low current. The RF envelope detector of on body electronics 1110 may operate in two modes - a desensitized mode where it is responsive to received signals of less than about 1 inch, and normal operating mode with normal signal sensitivity such that it is responsive to receives signals at a distance of about 3-12 inches.

During the initial pairing between display device 1120 and on body electronics 1110, in some embodiments, display device 1120 sends its identification information such as, for example, 4 bytes of display device ID which may include its serial number. On body electronics 1110 stores the received display device ID in one or more storage unit or memory component and subsequently includes the stored display device ID data in response packets or data provided to the display device 1120. In this manner, display device 1120 can discriminate detected data packets from on body electronics 1110 to determine that the received or detected data packets originated from the paired or correct on body electronics 1110. The pairing routine based on the display device ID in some embodiments avoids potential collision between multiple devices, especially in the cases where on body electronics 1110 does not selectively provide the analyte related data to a particular display device, but rather, provide to any display device within range and/or broadcast the data packet to any display device in communication range.

In some embodiments, the payload size from display device 1120 to on body electronics 1110 is 12 bytes, which includes 4 bytes of display device ID, 4 bytes of on body device ID, one byte of command data, one byte of spare data space, and two bytes for CRC (cyclic redundancy check) for error detection.

After pairing is complete, when display device 1120 queries on body electronics 1110 for real time monitored analyte information and/or logged or stored analyte data, in some embodiments, the responsive data packet transmitted to display device 1120 includes a total of 418 bytes that includes 34 bytes of status information, time information and calibration data, 96 bytes of the most recent 16 one-minute glucose data points, and 288 bytes of the most recent 15 minute interval glucose data over the 12 hour period. Depending upon the size or capacity of the memory or storage unit of on body electronics 1110, data stored and subsequently provided to the display device 1120 may have a different time resolution and/or span a longer or shorter time period. For example, with a larger data buffer, glucose related data provided to the display device 1120 may include glucose data over a 24 hour time period at 15 minute sampling intervals, 10 minute sampling intervals, 5 minute sampling intervals, or one minute sampling interval. Further, the determined variation in the monitored analyte level illustrating historical trend of the monitored analyte level may be processed and/or determined by the on body electronics 1110, or alternatively or in addition to, the stored data may be provided to the display device 1120 which may then determine the trend information of the monitored analyte level based on the received data packets.

The size of the data packets provided to display device 1120 from on body electronics 1110 may also vary depending upon the communication protocol and/or the underlying data transmission frequency - whether using a 433 MHz, a 13.56 MHz, or 2.45GHz in addition to other parameters such as, for example, the presence of data processing devices such as a processor or processing circuitry (*e.g.,* central processing unit CPU) in on body electronics 1110, in addition to the ASIC state machine, size of the data buffer and/or memory, and the like.

In some embodiments, upon successful activation of on body electronics 1110 and pairing with display device 1120, control unit of display device 1120 may be programmed to generate and output one or more visual, audible and/or haptic notifications to output to the user on display 1122, or on the user interface of display device 1120. In some embodiments, only one display device can pair with one on body electronics at one time. Alternatively, in some embodiments, one display device may be configured to pair with multiple on body electronics at the same time.

Once paired, display 1122 of display device 1120, for example, outputs, under the control of the processor of display device 1120, the remaining operational life of the analyte sensor 1101 in user. Furthermore, as the end of sensor life approaches, display device may be configured to output notifications to alert the user of the approaching end of sensor life. The schedule for such notification may be programmed or programmable by the user and executed by the processor of the display device.

Referring again to FIG. 21, in some embodiments, analyte monitoring system 1100 may store the historical analyte data along with a date and/or time stamp and/or and contemporaneous temperature measurement, in memory, such as a memory configured as a data logger as described above. In some embodiments, analyte data is stored at the frequency of about once per minute, or about once every ten minutes, or about once an hour, etc. Data logger embodiments may store historical analyte data for a predetermined period of time, *e.g.,* a duration specified by a physician, for example, *e.g.,* about 1 day to about 1 month or more, *e.g.,* about 3 days or more, *e.g.,* about 5 days or more, *e.g.,* about 7 days or more, *e.g.,* about 2 weeks or more, *e.g.,* about 1 month or more.

Other durations of time may be suitable, depending on the clinical significance of the data being observed. The analyte monitoring system 1100 may display the analyte readings to the subject during the monitoring period. In some embodiments, no data is displayed to the subject. Optionally, the data logger can transmit the historical analyte data to a receiving device disposed adjacent, *e.g.,* in close proximity to the data logger. For example, a receiving device may be configured to communicate with the data logger using a transmission protocol operative at low power over distances of a fraction of an inch to about several feet. For example, and without limitation, such close proximity protocols include Certified Wireless USB^{™}, TransferJet^{™}, Bluetooth^{®} (IEEE 802.15.1), WiFi^{™} (IEEE 802.11), ZigBee^{®} (IEEE 802.15.4-2006), Wibree^{™}, or the like.

The historical analyte data set may be analyzed using various diagnostic approaches. For example, the historical analyte data taken over several days may be correlated to the same date/and or time. The historical analyte data may be correlated to meal times. For example, data could take into account breakfast, lunch, and dinner. Data analysis for each meal could include some pre-prandial time (*e.g.,* 1 or 2 hours) and some post-prandial time (*e.g.,* 1-4 hours). Such an approach eliminates apparent glucose variability due to variability in the timing of meals alone. Analyte data parameters may be determined based upon the rate of change of one or more analyte levels. In some embodiments, an analyte data parameter may be determined concerning whether a threshold relating to an analyte value is exceeded, *e.g.,* a hyper-or hypoglycemia condition, the percentage of time in which the threshold is exceeded, or the duration of time in which the threshold is exceeded.

The analyte data parameters may be computed by a processor or processing circuitry executing a program stored in a memory. In some embodiments, the processor executing the program stored in the memory is provided in data processing module 1160 (FIG. 21). In some embodiments, the processor executing the program stored in the memory is provided in display device 1120. An example technique for analyzing data is the applied ambulatory glucose profile (AGP) analysis technique. Additional detailed descriptions are provided in U.S. Patent Nos. 5,262,035; 5,264,104; 5,262,305; 5,320,715; 5,593,852; 6,175,752; 6,650,471; 6,746, 582, 6,284,478, 7,299,082, and in U.S. Patent Application Nos. 10/745,878; 11/060,365, the disclosures of all of which are incorporated herein by reference in their entireties for all purposes.

As described above, in certain aspects of the present disclosure, discrete glucose measurement data may be acquired on-demand or upon request from the display device, where the glucose measurement is obtained from an in vivo glucose sensor transcutaneously positioned under the skin layer of a user, and further having a portion of the sensor maintained in fluid contact with the bodily fluid under the skin layer. Accordingly, in aspects of the present disclosure, the user of the analyte monitoring system may conveniently determine real time glucose information at any time, using the RFID communication protocol as described above.

In one aspect, the integrated assembly including the on body electronics and the insertion device may be sterilized and packaged as one single device and provided to the user. Furthermore, during manufacturing, the insertion device assembly may be terminal packaged providing cost savings and avoiding the use of, for example, costly thermoformed tray or foil seal. In addition, the insertion device may include an end cap that is rotatably coupled to the insertion device body, and which provides a safe and sterile environment (and avoid the use of desiccants for the sensor) for the sensor provided within the insertion device along with the integrated assembly. Also, the insertion device sealed with the end cap may be configured to retain the sensor within the housing from significant movement during shipping such that the sensor position relative to the integrated assembly and the insertion device is maintained from manufacturing, assembly and shipping, until the device is ready for use by the user.

### Embodiments disclosed herein include:

**Embodiment A:** A method comprising: displaying an analyte monitoring scan display window on a computing device, the analyte monitoring scan display window including an add note button; transitioning to an input display window on the computing device upon actuating the add note button, the input display window listing a limited number of user inputs associated with a sensor user's lifestyle events at a specific date and time; selecting one or more of the limited number of user inputs, the input display window configured for inputting information associated with the one or more selected user inputs; receiving into the input display window an input of information associated with the one or more selected user inputs; and displaying a selectable symbol correlating to a summary of the input of information on an analyte monitoring daily display window on the computing device at the specific date and time, wherein selecting the selectable symbol displays a pop-up display window on the computing device displaying the summary of the input of information overlaid upon the analyte monitoring daily display window.

**Embodiment B:** A system comprising: a computing device having a display screen configured to display a plurality of display windows comprising: an analyte monitoring scan display window including an add note button; an input display window listing a limited number of user inputs associated with a sensor user's lifestyle events at a specific date and time and configured for input of information associated with one or more selected user inputs; an analyte monitoring daily display window configured for displaying a selectable symbol correlating to a summary of the input of information at the specific date and time; and a pop-up display window that displays the summary of the input of information upon selecting the selectable symbol, wherein the pop-up display window is overlaid upon the analyte monitoring daily display window; and an analyte monitoring sensor communicably coupled to the computing device.

**Embodiment C:** A computing device having a display screen configured to display a plurality of display windows comprising: an analyte monitoring scan display window including an add note button; an input display window listing a limited number of user inputs associated with a sensor user's lifestyle at a specific date and time and configured for input of information associated with one or more selected user inputs; an analyte monitoring daily display window configured for displaying a selectable symbol correlating to a summary of the input of information at the specific date and time; and a pop-up display window that displays the summary of the input of information upon selecting the selectable symbol, wherein the pop-up display window is overlaid upon the analyte monitoring daily display window.

**Embodiment D:** A method comprising: displaying a menu display window of a computing device listing a limited number of user selectable buttons, including an event log button; and transitioning to an event log display window of the computing device upon selecting the event log button, wherein the event log display window displays one or more events associated with an analyte monitoring sensor at a specific date and time.

**Embodiment E:** A system comprising: a computing device having a display screen configured to display a plurality of display windows comprising: a menu display window listing a limited number of user selectable buttons including an event log button, an event log display window that displays one or more events associated with an analyte monitoring sensor at a specific date and time; and an analyte monitoring sensor communicably coupled to an analyte monitoring sensor.

**Embodiment F:** A computing device having a display screen configured to display a plurality of display windows comprising: a menu display window listing a limited number of user selectable buttons including an event log button, an event log display window that displays one or more events associated with an analyte monitoring sensor at a specific date and time.

Each of embodiments A, B, and C may have one or more of the following additional elements in any combination:
Element 1: Wherein the computing device is communicably coupled to an analyte monitoring sensor.
Element 2: Wherein the computing device is communicably coupled to a glucose monitoring sensor.
Element 3: Wherein the summary of the input of information is linked with an analyte measurement at the specific date and time.
Element 4: Wherein the pop-up display window further comprises a selectable edit button.
Element 5: Wherein the limited number of user inputs is selected from the group consisting of food, rapid-acting insulin, fast-acting insulin, exercise, comments, and any combination thereof.
Element 6: Wherein the analyte monitoring scan display window displays a graphical representation of an analyte concentration.
Element 7: Wherein the analyte monitoring scan display window displays a graphical representation of a glucose concentration.
Element 8: Wherein the analyte monitoring daily display window displays a graphical representation of an analyte concentration.
Element 9: Wherein the analyte monitoring daily display window displays a graphical representation of a glucose concentration.
Element 10: Further comprising closing the pop-up display window.
Element 11: Wherein the computing device is communicably coupled to an analyte monitoring sensor, and the limited number of user inputs associated with a sensor user's lifestyle events are dynamic based on analyte measurements from the analyte monitoring sensor.

By way of non-limiting example, exemplary combinations applicable to A, B, and C include, but are not limited to: any combination of 1-11, including each of 1-11, without limitation; 1 and 2; 1 and 3; 1 and 4; 1 and 5; 1 and 6; 1 and 7; 1 and 8; 1 and 9; 1 and 10; 1 and 11; 2 and 3; 2 and 4; 2 and 5; 2 and 6; 2 and 7; 2 and 8; 2 and 9; 2 and 10; 2 and 11; 3 and 4; 3 and 5; 3 and 6; 3 and 7; 3 and 8; 3 and 9; 3 and 10; 3 and 11; 4 and 5; 4 and 6; 4 and 7; 4 and 8; 4 and 9; 4 and 10; 4 and 11; 5 and 6; 5 and 7; 5 and 8; 5 and 9; 5 and 10; 5 and 11; 6 and 7; 6 and 8; 6 and 9; 6 and 10; 6 and 11; 7 and 8; 7 and 9; 7 and 10; 7 and 11; 8 and 9; 8 and 10; 8 and 11; 9 and 10; 9 and 11; 10 and 11; any of 1, 2, 3, 4, 5, 6, 7, 8, 9, 10, and 11 in any combination, without limitation.

Each of embodiments A, B, and C may have one or more of the following additional elements in any combination:
Element 12: Wherein the analyte monitoring sensor is a glucose monitoring sensor.
Element 13: Wherein limited number of user selectable buttons, including the event log button, further includes buttons selected from the group consisting of how to apply a sensor button, how to scan a sensor button, user's manual button, terms of use button, privacy notice button, and any combination thereof.
Element 14: Further comprising accessing the menu display window from a main menu display window.
Element 15: Further comprising accessing the menu display window from a main menu display window upon the user selecting a help button.
Element 16: Wherein the one or more events associated with the analyte monitoring sensor is selected from the group consisting of a scan error event, a sensor too cold event, a new sensor found event, and any combination thereof.
Element 17: Wherein the event log display window further comprises a send troubleshooting data button.
Element 18: Wherein the event log display window further comprises a send troubleshooting data button, further comprising sending information associated with the events to customer service personnel upon selecting the send troubleshooting data button.
Element 19: Wherein the event log display window displays the one or more events associated with the analyte monitoring sensor with an accompanying description of the one or more events.
Element 20: Wherein the event log display window displays the one or more events associated with the analyte monitoring sensor with an accompanying icon or symbol.
Element 21: Further comprising referencing on the event log display window a link to a user manual and associated page thereof associated with the one or more events.
Element 22: Further comprising providing on the event log display window remedial instructions associated with the one or more events.

By way of non-limiting example, exemplary combinations applicable to D, E, and F include, but are not limited to: any combination of 12-22, including each of 12-22, without limitation; 12 and 13; 12 and 14; 12 and 15; 12 and 16; 12 and 17; 12 and 18; 12 and 19; 12 and 20; 12 and 21; 12 and 22; 13 and 14; 13 and 15; 13 and 16; 13 and 17; 13 and 18; 13 and 19; 13 and 20; 13 and 21; 13 and 22; 14 and 4; 14 and 16; 14 and 17; 14 and 18; 14 and 19; 14 and 20; 14 and 21; 14 and 22; 15 and 16; 15 and 17; 15 and 18; 15 and 19; 15 and 20; 15 and 21; 15 and 22; 16 and 17; 16 and 18; 16 and 19; 16 and 20; 16 and 21; 16 and 22; 17 and 18; 17 and 19; 17 and 20; 17 and 21; 17 and 22; 18 and 19; 18 and 20; 18 and 21; 18 and 22; 19 and 20; 19 and 21; 19 and 22; 20 and 21; 20 and 22; 21 and 22; any of 12, 13, 14, 15, 16, 17, 18, 19, 20, 21, and 22 in any combination, without limitation.

Unless otherwise indicated, all numbers expressing quantities and the like in the present specification and associated claims are to be understood as being modified in all instances by the term "about." Accordingly, unless indicated to the contrary, the numerical parameters set forth in the following specification and attached claims are approximations that may vary depending upon the desired properties sought to be obtained by the embodiments of the present invention. At the very least, and not as an attempt to limit the application of the doctrine of equivalents to the scope of the claim, each numerical parameter should at least be construed in light of the number of reported significant digits and by applying ordinary rounding techniques.

One or more illustrative embodiments incorporating various features are presented herein. Not all features of a physical implementation are described or shown in this application for the sake of clarity. It is understood that in the development of a physical embodiment incorporating the embodiments of the present invention, numerous implementation-specific decisions must be made to achieve the developer's goals, such as compliance with system-related, business-related, government-related and other constraints, which vary by implementation and from time to time. While a developer's efforts might be time-consuming, such efforts would be, nevertheless, a routine undertaking for those of ordinary skill in the art and having benefit of this disclosure.

While various systems, tools and methods are described herein in terms of "comprising" various components or steps, the systems, tools and methods can also "consist essentially of" or "consist of" the various components and steps.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Therefore, the disclosed systems, tools and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems, tools and methods illustratively disclosed herein may suitably be practiced in the absence of any element that is not specifically disclosed herein and/or any optional element disclosed herein. While systems, tools and methods are described in terms of "comprising," "containing," or "including" various components or steps, the systems, tools and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

The disclosure of this application also contains the following numbered clauses:
1. A method comprising:
   displaying an analyte monitoring scan display window on a computing device, the analyte monitoring scan display window including an add note button;
   transitioning to an input display window on the computing device upon actuating the add note button, the input display window listing a limited number of user inputs associated with a sensor user's lifestyle events at a specific date and time;
   selecting one or more of the limited number of user inputs, the input display window configured for inputting information associated with the one or more selected user inputs;
   receiving into the input display window an input of information associated with the one or more selected user inputs; and
   displaying a selectable symbol correlating to a summary of the input of information on an analyte monitoring daily display window on the computing device at the specific date and time,
      wherein selecting the selectable symbol displays a pop-up display window on the computing device displaying the summary of the input of information overlaid upon the analyte monitoring daily display window.
2. The method of clause 1, wherein the computing device is communicably coupled to an analyte monitoring sensor.
3. The method of clause 1, wherein the computing device is communicably coupled to a glucose monitoring sensor.
4. The method of clause 1, wherein the summary of the input of information is linked with an analyte measurement at the specific date and time.
5. The method of clause 1, wherein the pop-up display window further comprises a selectable edit button.
6. The method of clause 1, wherein the limited number of user inputs is selected from the group consisting of food, rapid-acting insulin, fast-acting insulin, exercise, comments, and any combination thereof.
7. The method of clause 1, wherein the analyte monitoring scan display window displays a graphical representation of an analyte concentration.
8. The method of clause 1, wherein the analyte monitoring scan display window displays a graphical representation of a glucose concentration.
9. The method of clause 1, wherein the analyte monitoring daily display window displays a graphical representation of an analyte concentration.
10. The method of clause 1, wherein the analyte monitoring daily display window displays a graphical representation of a glucose concentration.
11. The method of clause 1, further comprising closing the pop-up display window.
12. The method of clause 1, wherein the computing device is communicably coupled to an analyte monitoring sensor, and the limited number of user inputs associated with a sensor user's lifestyle events are dynamic based on analyte measurements from the analyte monitoring sensor.
13. A system comprising:
   a computing device having a display screen configured to display a plurality of display windows comprising :
   an analyte monitoring scan display window including an add note button;
   an input display window listing a limited number of user inputs associated with a sensor user's lifestyle events at a specific date and time and configured for input of information associated with one or more selected user inputs;
   an analyte monitoring daily display window configured for displaying a selectable symbol correlating to a summary of the input of information at the specific date and time; and
   a pop-up display window that displays the summary of the input of information upon selecting the selectable symbol,
      wherein the pop-up display window is overlaid upon the analyte monitoring daily display window; and
   an analyte monitoring sensor communicably coupled to the computing device.
14. The system of clause 13, wherein the computing device is communicably coupled to an analyte monitoring sensor.
15. The system of clause 13, wherein the computing device is communicably coupled to a glucose monitoring sensor.
16. The system of clause 13, wherein the summary of the input of information is linked with an analyte measurement at the specific date and time.
17. The system of clause 13, wherein the pop-up display window further comprises a selectable edit button.
18. The system of clause 13, wherein the limited number of user inputs is selected from the group consisting of food, rapid-acting insulin, fast-acting insulin, exercise, comments, and any combination thereof.
19. The system of clause 13, wherein the analyte monitoring scan display window displays a graphical representation of an analyte concentration.
20. The system of clause 13, wherein the analyte monitoring daily display window displays a graphical representation of an analyte concentration.
21. A computing device having a display screen configured to display a plurality of display windows comprising:
   an analyte monitoring scan display window including an add note button;
   an input display window listing a limited number of user inputs associated with a sensor user's lifestyle at a specific date and time and configured for input of information associated with one or more selected user inputs;
   an analyte monitoring daily display window configured for displaying a selectable symbol correlating to a summary of the input of information at the specific date and time; and
   a pop-up display window that displays the summary of the input of information upon selecting the selectable symbol,
      wherein the pop-up display window is overlaid upon the analyte monitoring daily display window.
22. A method comprising:
   displaying a menu display window of a computing device listing a limited number of user selectable buttons, including an event log button; and
   transitioning to an event log display window of the computing device upon selecting the event log button,
      wherein the event log display window displays one or more events associated with an analyte monitoring sensor at a specific date and time.
23. The method of clause 22, wherein the analyte monitoring sensor is a glucose monitoring sensor.
24. The method of clause 22, wherein limited number of user selectable buttons, including the event log button, further includes buttons selected from the group consisting of how to apply a sensor button, how to scan a sensor button, user's manual button, terms of use button, privacy notice button, and any combination thereof.
25. The method of clause 22, further comprising accessing the menu display window from a main menu display window.
26. The method of clause 22, further comprising accessing the menu display window from a main menu display window upon the user selecting a help button.
27. The method of clause 22, wherein the one or more events associated with the analyte monitoring sensor is selected from the group consisting of a scan error event, a sensor too cold event, a new sensor found event, and any combination thereof.
28. The method of clause 22, wherein the event log display window further comprises a send troubleshooting data button.
29. The method of clause 22, wherein the event log display window further comprises a send troubleshooting data button, further comprising sending information associated with the events to customer service personnel upon selecting the send troubleshooting data button.
30. The method of clause 22, wherein the event log display window displays the one or more events associated with the analyte monitoring sensor with an accompanying description of the one or more events.
31. The method of clause 22, wherein the event log display window displays the one or more events associated with the analyte monitoring sensor with an accompanying icon or symbol.
32. The method of clause 22, further comprising referencing on the event log display window a link to a user manual and associated page thereof associated with the one or more events.
33. The method of clause 22, further comprising providing on the event log display window remedial instructions associated with the one or more events.
34. A system comprising:
   a computing device having a display screen configured to display a plurality of display windows comprising:
      a menu display window listing a limited number of user selectable buttons including an event log button,
      an event log display window that displays one or more events associated with an analyte monitoring sensor at a specific date and time; and
   an analyte monitoring sensor communicably coupled to an analyte monitoring sensor.
35. The system of clause 34, wherein the analyte monitoring sensor is a glucose monitoring sensor.
36. The system of clause 34, wherein limited number of user selectable buttons including the event log button further includes buttons selected from the group consisting of how to apply a sensor button, how to scan a sensor button, user's manual button, terms of use button, privacy notice button, and any combination thereof.
37. The system of clause 34, wherein the menu display window is accessed from a main menu display window.
38. The system of clause 34, wherein the menu display window is accessed from a main menu display window upon the user selecting a help button.
39. The system of clause 34, wherein the one or more events associated with the analyte monitoring sensor is selected from the group consisting of a scan error event, a sensor too cold event, a new sensor found event, and any combination thereof.
40. The system of clause 34, wherein the event log display window further comprises a send troubleshooting data button.
41. The system of clause 34, wherein the event log display window further comprises a send troubleshooting data button, an information associated with the events is sent to customer service personnel upon selecting the send troubleshooting data button.
42. The system of clause 34, wherein the event log display window displays the one or more events associated with the analyte monitoring sensor with an accompanying description of the one or more events.
43. A computing device having a display screen configured to display a plurality of display windows comprising:
   a menu display window listing a limited number of user selectable buttons including an event log button,
   an event log display window that displays one or more events associated with an analyte monitoring sensor at a specific date and time.

## Claims

1. A method comprising:
determining, by a glucose monitoring sensor, glucose levels in a body and transmitting data associated with the glucose levels;
displaying, by a computing device in communication with the glucose monitoring sensor, the data associated with the glucose levels;
displaying a menu display window of the computing device listing a limited number of user selectable buttons, including an event log button; and
transitioning to an event log display window of the computing device upon selecting the event log button, wherein the event log display window displays one or more events associated with the glucose monitoring sensor at a specific date and time; and
providing on the event log display window remedial instructions associated with the one or more events.

2. The method of claim 1, wherein the limited number of user selectable buttons further comprises buttons selected from the group consisting of how to apply a sensor button, how to scan a sensor button, user's manual button, terms of use button, privacy notice button, and any combination thereof.

3. The method of claim 1, further comprising accessing the menu display window from a main menu display window.

4. The method of claim 1, wherein the one or more events associated with the glucose monitoring sensor is selected from the group consisting of a scan error event, a sensor too cold event, a new sensor found event, and any combination thereof.

5. The method of claim 1, wherein the event log display window further comprises a send troubleshooting data button.

6. The method of claim 1, wherein the event log display window further comprises a send troubleshooting data button, further comprising sending information associated with the events to customer service personnel upon selecting the send troubleshooting data button.

7. The method of claim 1, wherein the event log display window displays the one or more events associated with the glucose monitoring sensor with an accompanying description of the one or more events.

8. The method of claim 1, wherein the event log display window displays the one or more events associated with the glucose monitoring sensor with an accompanying icon or symbol.

9. The method of claim 1, further comprising referencing on the event log display window a link to a user manual and associated page thereof associated with the one or more events.

10. A system comprising:
a glucose monitoring sensor communicably coupled to a computing device, wherein the computing device is configured to display data associated with glucose levels, and wherein the computing device comprises a display screen configured to display a plurality of display windows comprising:
a menu display window listing a limited number of user selectable buttons including an event log button; and
an event log display window that displays one or more events associated with the glucose monitoring sensor at a specific date and time when the event log button is selected, wherein the event log display window further displays remedial instructions associated with the one or more events.

11. The system of claim 10, wherein the limited number of user selectable buttons further comprises buttons selected from the group consisting of how to apply a sensor button, how to scan a sensor button, user's manual button, terms of use button, privacy notice button, and any combination thereof.

12. The system of claim 10, wherein the menu display window is accessed from a main menu display window.

13. The system of claim 10, wherein the one or more events associated with the glucose monitoring sensor is selected from the group consisting of a scan error event, a sensor too cold event, a new sensor found event, and any combination thereof.

14. The system of claim 10, wherein the event log display window further comprises a send troubleshooting data button, and optionally wherein information associated with the events is sent to customer service personnel upon selecting the send troubleshooting data button.

15. The system of claim 10, wherein the event log display window displays the one or more events associated with the glucose monitoring sensor with an accompanying description of the one or more events.
